# EUROPÄISCHE PATENTSCHRIFT

(11) **EP 2 079 757 B1**
(45) Veröffentlichungstag und Bekanntmachung des Hinweises auf die Patenterteilung: **17.12.2014**
(21) Anmeldenummer: 07819248.1
(22) Anmeldetag: 23.10.2007
(51) Int. Cl.: C07K 14/00, C07K 5/06

(54) **VERFAHREN ZUR SELEKTIVEN LOKALISIERUNG VON WIRKSTOFFEN AN UND IN MITOCHONDRIEN UND ENTSPRECHENDE WIRKSTOFFE**
PROCESS FOR SELECTIVELY LOCALIZING ACTIVE INGREDIENTS ON AND IN MITOCHONDRIA AND CORRESPONDING ACTIVE INGREDIENTS
PROCÉDÉ DE LOCALISATION SÉLECTIVE DE SUBSTANCES ACTIVES SUR ET DANS DES MITOCHONDRIES ET SUBSTANCES ACTIVES CORRESPONDANTES

(30) Priorität: 24.10.2006 DE 102006050091
(43) Veröffentlichungstag der Anmeldung: 22.07.2009
(73) Patentinhaber: Ugichem Gesellschaft Für Organische Chemie MbH, 6020 Innsbruck (AT)
(72) Erfinder: LINDHORST, Thomas, 6020 Innsbruck (AT); WERNER, Birgit, 6020 Innsbruck (AT); PIPER, Stefan, 79592 Fischingen (DE)
(74) Vertreter: Forstmeyer, Dietmar
(86) Internationale Anmeldenummer: PCT/EP2007/009187
(87) Internationale Veröffentlichungsnummer: WO 2008/049583

(56) Entgegenhaltungen:
- WO-A-00/52038
- WO-A-99/37294
- WO-A-2008/009470
- US-A1- 2005 031 651
- LIGERET HEIDI ET AL: "Effects of curcumin and curcumin derivatives on mitochondrial permeability transition pore." FREE RADICAL BIOLOGY & MEDICINE, Bd. 36, Nr. 7, 1. April 2004 (2004-04-01), Seiten 919-929, XP002470905 ISSN: 0891-5849
- FRANCES H BLAIKIE ET AL: "Targeting Dinitrophenol to Mitochondria: Limitations to the Development of a Self-limiting Mitochondrial Protonophore" BIOSCIENCE REPORTS, KLUWER ACADEMIC PUBLISHERS-PLENUM PUBLISHERS, NE, Bd. 26, Nr. 3, 19. Juli 2006 (2006-07-19), Seiten 231-243, XP019391966 ISSN: 1573-4935
- WEISSIG V: "Mitochondrial-targeted drug and DNA delivery" CRITICAL REVIEWS IN THERAPEUTIC DRUG CARRIER SYSTEMS 2003 UNITED STATES, Bd. 20, Nr. 1, 2003, Seiten 1-62, XP009096494 ISSN: 0743-4863
- RAZUMIENE, J. ET AL: "New bioorganometallic ferrocene derivatives as efficient mediators for glucose and ethanol biosensors based on PQQ-dependent dehydrogenases" JOURNAL OF ORGANOMETALLIC CHEMISTRY, Bd. 668, 2003, Seiten 83-90, XP002513485
- JEONG T-S ET AL: "Novel 3,5-diaryl pyrazolines and pyrazole as low-density lipoprotein (LDL) oxidation inhibitor" BIOORGANIC & MEDICINAL CHEMISTRY LETTERS, PERGAMON, ELSEVIER SCIENCE, GB, Bd. 14, Nr. 11, 7. Juni 2004 (2004-06-07), Seiten 2719-2723, XP004841276 ISSN: 0960-894X
- JAUSLIN M L ET AL: "Mitochondria-targeted antioxidants protect Friedreich Ataxia fibroblasts from endogenous oxidative stress more effectively than untargeted antioxidants" FASEB JOURNAL, FED. OF AMERICAN SOC. FOR EXPERIMENTAL BIOLOGY, BETHESDA, MD, US, Bd. 17, Nr. 13, 1. Oktober 2003 (2003-10-01), Seiten 1972-1974, XP002409006 ISSN: 0892-6638
- SHEU ET AL: "Targeting antioxidants to mitochondria: A new therapeutic direction" BIOCHIMICA ET BIOPHYSICA ACTA. MOLECULAR BASIS OF DISEASE, AMSTERDAM, NL, Bd. 1762, Nr. 2, 1. Februar 2006 (2006-02-01), Seiten 256-265, XP005230510 ISSN: 0925-4439
- REDDY P H: "Mitochondrial oxidative damage in aging and Alzheimer's disease: Implications for mitochondrially targeted antioxidant therapeutics" JOURNAL OF BIOMEDICINE AND BIOTECHNOLOGY 2006 US, Bd. 2006, 2006, XP002513486 ISSN: 1110-7243 1110-7251

## Beschreibung

Die vorliegende Erfindung betrifft neue Verfahren zur selektiven Lokalisierung von Wirkstoffen sowohl an als auch in Mitochondrien innerhalb von lebenden Zellen sowie entsprechende Wirkstoffe, die ohne weitere Hilfsmittel durch die Zellmembran in Zellen eindringen und dort selektiv sowohl an als auch in Mitochondrien lokalisiert werden.

Lokalisierung von Wirkstoffen an bzw. in Mitochondrien bedeutet in der gesamten Anmeldung die Anreicherung von Wirkstoffen an bzw. in Mitochondrien. Der Begriff "lokalisiert" an bzw. in Mitochondrien bedeutet in der gesamten Anmeldung "angereichert" an bzw. in Mitochondrien.

Mitochondrien sind semiautonome Organellen der Zelle. Sie besitzen ein eigenes Genom (mtDNA), das neben dem Kerngenom für einen Teil ihrer Proteine kodiert. Die mitochondrial kodierten Proteine werden auch mitochondrial transkribiert, translatiert und synthetisiert. Wichtige Stoffwechselwege in den Mitochondrien dienen der Energiegewinnung und sind somit essentiell für die Vitalität der Zelle.

Mitochondriale Krankheiten umfassen beispielsweise zahlreiche Erbkrankheiten, Krebs, Diabetes, Parkinson und Arteriosklerose. Mitochondriale Stoffwechselstörungen werden unter anderem auch für Phänomene des Alterns, beispielsweise Schwerhörigkeit oder das Nachlassen der Sehkraft verantwortlich gemacht. Phänomene des Alterns werden beispielsweise auch auf Mutationen bzw. Deletionen der mitochondrialen DNA zurückgeführt.

("Mitochondria as targets for detection and treatment of cancer", Josephine S. Modica-Napolitano, Keshav K. Singh, expert reviews in molecular medicine, (02)00495-3a.pdf (short code: txt001ksb); 11 April 2002, ISSN 1462-3994 ©2002 Cambridge University Press. "Mitochondrial defects in cancer", Jennifer S Carew, Peng Huang, Molecular Cancer, 2002, I:9. G. A. Cortopassi, Aliu Wong, Biochinica et Biophysica Acta (BBA)-Bioenergetics, 1999, 1410 (2), 183-193.).

Die den mitochondrialen Krankheiten zugrunde liegenden Gendefekte reichen von sporadisch auftretenden und auch rein maternal vererbten Punkt- und Längenmutationen der mtDNA bis hin zu autosomal dominant bzw. rezessiv vererbte Formen bei Veränderungen im Kerngenom. Außerdem wird diskutiert, dass Veränderungen in der mtDNA auch bei polygenen Erkrankungen mit komplizierten Erbgängen eine Rolle spielen.

Besonderheiten dieser Erkrankungen sind die Vielfältigkeit ihrer klinischen Bilder, die Komplexität der Diagnostik und die bisher nur beschränkt vorhandenen Therapieansätze.

Somit ist die Erforschung und Diagnose bestimmter Eigenschaften der Mitochondrien, wie zum Beispiel Mutationen der mitochondrialen DNA oder mitochondriale Stoffwechselstörungen, eine wichtige Voraussetzung in der Entwicklung entsprechender Wirkstoffe gegen mitochondriale Krankheiten.

Die selektive Lokalisierung von Wirkstoffen an Mitochondrien innerhalb von Zellen ist dabei ebenfalls ein wichtiger Aspekt, da dadurch eine hohe lokale Wirkstoff-Konzentration an den Mitochondrien erzeugt wird, die schließlich zu einem Import der Wirkstoffe in die Mitochondrien führt.

Blaikie et al., "Targeting Dinitrophenol to Mitochondria: Limitations to the Development of a Self-limiting Mitochondrial Protonophore", Bioscience Reports, Bd. 26 (3), Seiten 231-243 (2006) berichten über die Herstellung der Verbindung MitoDNP (3-(3,5-Dinitro-4-hydroxyphenyl)propyl-triphenylphosphonium-methansulfonat), die sich in Mitochondrien anreichert. Ferner wurde festgestellt, dass MitoDNP in den Mitochondrien verbleibt und das Protonen-Membran-Potenzial nicht entkoppelt.

Im Übersichtsartikel von Weissig, "Mitochondrial-targeted drug and DNA delivery", Critical Reviews in Therapeutic Drug Carrier Systems, Bd. 20 (1), Seiten 1-62 (2003) werden verschiedene Strategien zum gezielten Targeting von Mitochondrien sowie mögliche therapeutische Anwendungen diskutiert - unter anderem die Zuführung von Oligonukleotiden, Peptidnukleinsäuren (PNAs), oder von Plasmid-DNA sowohl an als auch in Mitochondrien. Die darin beschriebenen importierten Nukleinsäuren zeigen aber innerhalb der mitochondrialen Matrix keine Wirksamkeit.

Mitochondriale Wirkstoffe sind Substanzen, die eine Wirksamkeit sowohl an als auch in Mitochondrien erzielen, wie zum Beispiel Wirksamkeit in der Behandlung von mitochondrialen Krankheiten oder Wirksamkeit in Bezug auf diagnostische Verfahren sowohl an als auch in Mitochondrien.

Eine Aufgabe der vorliegenden Erfindung ist es, Antisense-Wirkstoffe zur Verfügung zu stellen, die ohne weitere Hilfsmittel durch die Zellmembran in Zellen eindringen und dort selektiv sowohl an als auch in Mitochondrien lokalisiert werden, um in Mitochondrien einen Antisense- oder einen antigenomischen Effekt zu erzeugen.

Diese Aufgabe wird gelöst durch Verbindungen der allgemeinen Formel I: 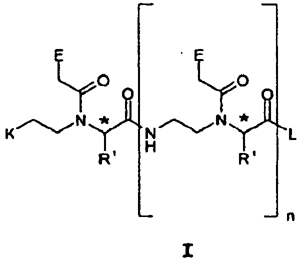 worin
n eine ganze Zahl von 0 bis 35 ist, bevorzugt 1 bis 28, stärker bevorzugt 9 bis 28, am stärksten bevorzugt 13 bis 20.

Die Reste K, L oder R¹ sind unabhängig voneinander mit mindestens einem Monohydroxy-mononitro-phenyl-Rest, bevorzugt ein 4-Hydroxy-3-nitro-phenyl-Rest, weiterhin bevorzugt ein 4-Hydroxy-2-nitro-phenyl-Rest, weiterhin bevorzugt ein 3-Hydroxy-6-nitro-phenyl-Rest, oder einem Monohydroxy-dinitro-phenyl-Rest, bevorzugt ein 3,5-Dinitro-4-hydroxy-phenyl-Rest, weiterhin bevorzugt ein 2,5-Dinitro-4-hydroxy-phenyl-Rest, weiterhin bevorzugt ein 2,4-Dinitro-5-hydroxy-phenyl-Rest, substituiert, wobei die Verknüpfungsposition des Phenyl-Rests mit den Resten K, L oder R¹ als Position 1 definiert ist und der Phenyl-Rest zusätzlich mit einem/einer oder mehreren Fluor-, Chlor-, Brom- oder Jodatomen oder -COOH, COOR⁸, -CSOH, CSOR⁸, -COSH, COSR⁸, -CONH₂, -CONHR⁹,-COR¹⁰R¹¹, -OH, -OR⁸, -SH, -SR⁸, -NH₂, -NHR⁹, -NR¹⁰R¹¹, -NR¹²NOH, -NOR¹³, Phosphonsäureester- oder Phosphonsäure-Funktionen oder C₁-C₆ Alkyl-, C₂-C₆ Alkenyl-, C₂-C₆ Alkinyl-, C₁-C₆ Heteroalkyl-, C₃-C₁₀ Cycloalkyl-, C₃-C₁₀ Heterocycloalkyl-, C₆-C₁₀ Aryl-, C₅-C₉ Heteroaryl-, C₇-C₁₂ Aralkyl- oder C₂-C₁₁ Heteroaralkyl-Gruppen substituiert sein kann, wobei R⁸, R⁹, R¹⁰, R¹¹, R¹² und R¹³ unabhängig voneinander C₁-C₆ Alkylreste sind.

E ist unabhängig voneinander ein H-Atom, ein substituierter oder unsubstituierter Phenylrest, ein substituierter oder unsubstituierter Heterocyclus, eine gegebenenfalls mit Schutzgruppen substituierte Nukleobase, z.B. eine in der Natur vorkommende oder nicht in der Natur vorkommende Nukleobase, oder ein DNA-Intercalator.

Bevorzugt ist jedes E unabhängig voneinander ein Adeninyl-, Cytosinyl-, Pseudoisocytosinyl-, Guaninyl-, Thyminyl-, Uracilyl- oder Phenyl-Rest.

Jeder Rest R¹ ist unabhängig voneinander ein H-Atom oder ein gegebenenfalls substituierter Alkyl-, Alkenyl-, Alkylaryl-, Aryl-, heterocyclischer oder alicyclischer Rest mit bis zu 20 C-Atomen, wobei mindestens ein Rest R¹ kein H-Atom und mit einer oder mehreren Phosphonsäureester- oder Phosphonsäure-Funktionen substituiert ist.

Wenn der Rest R¹ nicht mit einer oder mehreren Phosphonsäureester- oder Phosphonsäure-Funktionen substituiert ist, kann er z.B. auch unabhängig voneinander eine oder mehrere Seitenketten einer natürlichen oder nichtnatürlichen Aminosäure aufweisen, bevorzugt einen gegebenenfalls substituierten Alkyl-, Alkenyl-, Alkylaryl-, Aryl-, heterocyclischen oder alicyclischen Rest mit bis zu 20 C-Atomen.

Bevorzugt umfasst jeder Rest R¹ unabhängig voneinander 1, 2, 3, 4, 5, 6, 7, 8, 9 oder 10 C-Atome.

Jeder Rest R¹ kann unabhängig voneinander verzweigt oder unverzweigt sein.

Der Ausdruck "gegebenenfalls substituiert" bezieht sich in der gesamten Anmeldung auf Gruppen, in denen ein oder mehrere Wasserstoffatome durch Fluor-, Chlor-, Brom- oder Jodatome oder-COOH, COOR⁸, -CSOH, CSOR⁸, -COSH, COSR⁸, -CONH₂, -CONHR⁹, -COR¹⁰R¹¹, -OH, -OR⁸, =O, -SH, -SR⁸, =S, -NH₂, =NH, -NHR⁹, -NR¹⁰R¹¹, -NR¹²NOH, -NOR¹³ oder NO₂-Gruppen, Phosphonsäureester- oder Phosphonsäure-Funktionen ersetzt sind. Dieser Ausdruck bezieht sich weiterhin auf Gruppen, die mit unsubstituierten C₁-C₆ Alkyl-, C₂-C₆ Alkenyl-, C₂-C₆ Alkinyl-, C₁-C₆ Heteroalkyl-, C₃-C₁₀ Cycloalkyl-, C₂-C₉ Heterocycloalkyl-, C₆-C₁₀ Aryl-, C₅-C₉ Heteroaryl-, C₇-C₁₂ Aralkyl- oder C₂-C₁₁ Heteroaralkyl-Gruppen substituiert sind, wobei R⁸, R⁹, R¹⁰, R¹¹, R¹² und R¹³ unabhängig voneinander C₁-C₆ Alkylreste sind.

Phosphonsäureester-Funktionen können zum Beispiel die Formel -P(=O)(OV)₂ oder -P(=O)(OV)(OH) aufweisen. Dabei kann jedes V unabhängig voneinander ein unsubstituierter Alkyl-, Alkenyl-, Alkylaryl-, Aryl-, oder alicyclischer Rest mit bis zu 20 C-Atomen, stärker bevorzugt mit bis 7 Atomen und am stärksten bevorzugt ein Methyl-, Ethyl-, Cyclohexyl, oder Benzyl-Rest sein.

Bei den erfindungsgemäßen Verbindungen können die Phosphonsäure-Funktionen zum Beispiel die Formel -P(=O)(OH)₂ aufweisen.

Am stärksten bevorzugt wird jeder Rest R¹ unabhängig voneinander aus einer Gruppe der Formeln -(C₁-C₁₀)Alkyl-[P(=O)(O-V)₂] ausgewählt, wobei jedes V unabhängig voneinander ein H-Atom, ein Methyl-, Ethyl-, Cyclohexyl-, oder ein Benzyl-Rest ist.

K ist eine Gruppe der Formel -NR²R³, -N^{⊕}R²R³R⁹, -NR²(CO)R³ oder-NR²(CS)R³, wobei R², R³ und R⁴ unabhängig voneinander ein H-Atom, ein Alkyl-, Alkaryl-, Alkenyl, oder Alkinyl- Rest, Amino-Schutzgruppe, Reporterligand, Fluoreszenz-Marker, Intercalator, Chelator, Aminosäure, Peptid, Protein, Kohlenhydrat, Lipid, Steroid, Fettsäure, Oligonukleotid, Quantum-Dot, FRET-Quencher (Fluoreszenz-Resonanz-Energie-Transfer-Quencher) oder ein in Wasser lösliches oder unlösliches Polymer sind, wobei jeder der vorstehenden Reste gegebenenfalls substituiert sein kann. Bevorzugt ist K eine NH₂-Funktion, ein -NH(CO)CH₃-Rest, eine-NH (CO) - (C₁-C₁₀) Alkyl-Funktion, eine -NH (CO) - (C₁-C₁₀) Alkaryl-Funktion, eine -NH(CO)-(C₁-C₁₀)Alkenyl-Funktion, eine -NH(CO)-(C₁-C₁₀)Alkinyl-Funktion, eine Gruppe der Formel -NR²R³ oder -N^{⊕}R²R³R⁴ oder -NR²(CO)R³, wobei R², R³ und R⁴ unabhängig voneinander ein H-Atom, eine natürliche oder nicht-natürliche Aminosäure, eine unsubstituierte oder mit Phosphonsäureester- oder Phosphonsäure-Funktionen substituierte Aminosäure, Peptid oder Alkyl-, Alkaryl-, Alkenyl, oder Alkinyl- Rest sind, wobei jeder der vorstehenden Reste gegebenenfalls substituiert sein kann.

L ist eine Gruppe der Formel -NR⁵R⁶, -NR⁵(CO)R⁶, -NR⁵(CS)R⁶, -OR⁷ oder -SR⁷ wobei R⁵ und R⁶ unabhängig voneinander ein H-Atom, ein Alkyl-, Alkaryl-, Alkenyl, oder Alkinyl- Rest, Reporterligand, Fluoreszenz-Marker, Intercalator, Chelator, Aminosäure, Aminosäureamid, Peptid, Peptidamid, Protein, Kohlenhydrat, Lipid, Steroid, Fettsäure, Oligonukleotid , Quantum-Dot, FRET-Quencher (Fluoreszenz-Resonanz-Energie-Transfer-Quencher) oder ein in Wasser lösliches oder ein unlösliches Polymer und R⁷ ein H-Atom, ein Alkylrest, Reporterligand, Fluoreszenz-Marker, Intercalator, Chelator, Aminosäure, Aminosäureamid, Peptid, Peptidamid, Protein, Kohlenhydrat, Lipid, Steroid, Fettsäure, Oligonukleotid, Quantum-Dot, FRET-Quencher oder ein in Wasser lösliches oder ein unlösliches Polymer ist, wobei jeder der vorstehenden Reste gegebenenfalls substituiert sein kann.

Bevorzugt ist L eine OH-Funktion, eine NH₂-Funktion, eine -NH-(C₁-C₁₀)Alkyl-Funktion, eine -NH-(C₁-C₁₀)Alkaryl-Funktion, eine -NH-(C₁-C₁₀) Alkenyl-Funktion, eine -NH-(C₁-C₁₀)Alkinyl-Funktion, eine natürliche oder nicht-natürliche Aminosäure, eine unsubstituierte oder mit Phosphonsäureester- oder Phosphonsäure-Funktionen substituierte Aminosäure-, Aminosäureamid-, Peptid- oder Peptidamid-Einheit, wobei jeder der vorstehenden Reste gegebenenfalls substituiert sein kann.

In der gesamten Anmeldung können Alkylreste vorzugsweise 1-6 C-Atome aufweisen, z. B. Methyl-, Ethyl-, Propyl- oder Butyl-Gruppen sein. Die Ausdrücke "Aralkyl", "Alkaryl" und "Arylalkyl" bedeuten in der gesamten Anmeldung eine Gruppe, die einen aliphatischen und einen aromatischen Teil aufweist.

Wenn R¹ kein H-Atom ist, entsteht durch die Bindung vom Rest R¹ an das Backbone der allgemeinen Verbindung I an der Verbindungsstelle ein asymmetrisches Zentrum (*). An jedem asymmetrischen Zentrum liegt demnach eine R-Konfiguration oder eine S-Konfiguration vor.

Dabei wird die Konfiguration vorzugsweise am asymmetrischen Zentrum analog den Cahn-Ingold-Prelog-Regeln definiert, mit der zusätzlichen Maßgabe, dass die Priorität der Liganden immer wie folgt definiert ist: Das Stickstoffatom am asymmetrischen Zentrum erhält immer die Priorität 1. Das Kohlenstoffatom der Carboxylgruppe am asymmetrischen Zentrum erhält immer die Priorität 2. Das Kohlenstoffatom des Rests R¹ am asymmetrischen Zentrum erhält immer die Priorität 3. Das Wasserstoffatom am asymmetrischen Zentrum erhält immer die Priorität 4.

Erfindungsgemäß weisen die Verbindungen der allgemeinen Formel I mindestens 1 asymmetrisches Zentrum auf, wobei mindestens ein Rest R¹ mit einer oder mehreren Phosphonsäureester- oder Phosphonsäure-Funktionen substituiert ist.

Gemäß einer weiteren bevorzugten Ausführungsform der Erfindung entspricht jeder zweite Rest R¹ unabhängig voneinander der Seitenkette einer natürlichen oder nichtnatürlichen Aminosäure, bevorzugt einem gegebenenfalls substituierten Alkyl-, Alkenyl-, Alkylaryl-, Aryl-, heterocyclischen oder alicyclischen Rest mit bis zu 20 C-Atomen und mindestens ein Rest R¹ ist ein gegebenenfalls substituierter Alkyl-, Alkenyl-, Alkylaryl-, Aryl- oder alicyclischer Rest mit bis zu 20 C-Atomen, der mit einer oder mehreren Phosphonsäureester- oder Phosphonsäure-Funktionen substituiert ist, wobei die übrigen Reste R¹ H-Atome sind.

Gemäß einer weiteren bevorzugten Ausführungsform der Erfindung entspricht jeder dritte Rest R¹ unabhängig voneinander der Seitenkette einer natürlichen oder nichtnatürlichen Aminosäure, bevorzugt einem gegebenenfalls substituierten Alkyl-, Alkenyl-, Alkylaryl-, Aryl-, heterocyclischen oder alicyclischen Rest mit bis zu 20 C-Atomen und mindestens ein Rest R¹ ist ein gegebenenfalls substituierter Alkyl-, Alkenyl-, Alkylaryl-, Aryl- oder alicyclischer Rest mit bis zu 20 C-Atomen und mit einer oder mehreren Phosphonsäureester- oder Phosphonsäure-Funktionen substituiert, wobei die übrigen Reste R¹ H-Atome sind.

Gemäß einer weiteren bevorzugten Ausführungsform der Erfindung entsprechen zwei, drei oder mehr benachbarte Reste R¹ unabhängig voneinander der Seitenkette einer natürlichen oder nichtnatürlichen Aminosäure, bevorzugt einem gegebenenfalls substituierten Alkyl-, Alkenyl-, Alkylaryl-, Aryl-, heterocyclischen oder alicyclischen Rest mit bis zu 20 C-Atomen und mindestens ein Rest R¹ ist ein gegebenenfalls substituierter Alkyl-, Alkenyl-, Alkylaryl-, Aryl- oder alicyclischer Rest mit bis zu 20 C-Atomen und mit einer oder mehreren Phosphonsäureester- oder Phosphonsäure-Funktionen substituiert, wobei die übrigen Reste R¹ H-Atome sind.

Gemäß einer weiteren bevorzugten Ausführungsform der Erfindung entspricht jedes R¹ unabhängig voneinander der Seitenkette einer natürlichen oder nichtnatürlichen Aminosäure, bevorzugt einem gegebenenfalls substituierten Alkyl-, Alkenyl-, Alkylaryl-, Aryl-, heterocyclischen oder alicyclischen Rest mit bis zu 20 C-Atomen und mindestens ein Rest R¹ ist ein gegebenenfalls substituierter Alkyl-, Alkenyl-, Alkylaryl-, Aryl- oder alicyclischer Rest mit bis zu 20 C-Atomen und mit einer oder mehreren Phosphonsäureester- oder Phosphonsäure-Funktionen substituiert.

Gemäß einer weiteren bevorzugten Ausführungsform der Erfindung weisen einer oder mehrere der Reste R¹ unabhängig voneinander mindestens eine Phosphonsäureester- oder Phosphonsäure-Funktion auf.

Gemäß weiterer bevorzugter Ausführungsformen der vorliegenden Erfindung gilt folgendes:
Sind in der Verbindung der allgemeinen Formel I mehr als ein asymmetrisches Zentrum und mehr als ein gegebenenfalls substitutierter Rest R¹ mit einer oder mehreren Phosphonsäureester- oder Phosphonsäure-Funktionen vorhanden, haben mindestens 50% der Anzahl der asymmetrischen Zentren, die Reste mit einer oder mehreren Phosphonsäureester- oder Phosphonsäure-Funktionen aufweisen, die R-Konfiguration, bevorzugt 66%, stärker bevorzugt 70%, stärker bevorzugt 75%, stärker bevorzugt 80%, stärker bevorzugt 85%, stärker bevorzugt 90%, stärker bevorzugt 95%, am stärksten bevorzugt 100%.

Gemäß alternativer bevorzugter Ausführungsformen der vorliegenden Erfindung gilt folgendes:
Sind in der Verbindung der allgemeinen Formel I mehr als ein asymmetrisches Zentrum und mehr als ein gegebenenfalls substitutierter Rest R¹ mit einer oder mehreren Phosphonsäureester- oder Phosphonsäure-Funktionen vorhanden, haben mindestens 50% der Anzahl der asymmetrischen Zentren, die Reste mit einer oder mehreren Phosphonsäureester- oder Phosphonsäure-Funktionen aufweisen, die S-Konfiguration, bevorzugt 66%, stärker bevorzugt 70%, stärker bevorzugt 75%, stärker bevorzugt 80%, stärker bevorzugt 85%, stärker bevorzugt 90%, stärker bevorzugt 95%, am stärksten bevorzugt 100%.

In einer weiteren Ausführungsform sind maximal 80% der Anzahl der Reste R¹ mit Phosphonsäureester- oder Phosphonsäure-Funktionen substituiert und die übrigen Reste R¹ sind H-Atome.

In einer weiteren Ausführungsform sind maximal 60% der Anzahl der Reste R¹ mit Phosphonsäureester- oder Phosphonsäure-Funktionen substituiert und die übrigen Reste R¹ sind H-Atome.

In einer weiteren Ausführungsform sind maximal 50% der Anzahl der Reste R¹ mit Phosphonsäureester- oder Phosphonsäure-Funktionen substituiert und die übrigen Reste R¹ sind H-Atome.

In einer weiteren Ausführungsform sind maximal 40% der Anzahl der Reste R¹ mit Phosphonsäureester- oder Phosphonsäure-Funktionen substituiert und die übrigen Reste R¹ sind H-Atome.

In einer weiteren Ausführungsform sind maximal 30% der Anzahl der Reste R¹ mit Phosphonsäureester- oder Phosphonsäure-Funktionen substituiert und die übrigen Reste R¹ sind H-Atome.

In einer weiteren Ausführungsform sind maximal 20% der Anzahl der Reste R¹ mit Phosphonsäureester- oder Phosphonsäure-Funktionen substituiert und die übrigen Reste R¹ sind H-Atome.

In einer weiteren Ausführungsform sind maximal 10% der Anzahl der Reste R¹ mit Phosphonsäureester- oder Phosphonsäure-Funktionen substituiert und die übrigen Reste R¹ sind H-Atome.

In einer weiteren Ausführungsform sind maximal 4% der Anzahl der Reste R¹ mit Phosphonsäureester- oder Phosphonsäure-Funktionen substituiert und die übrigen Reste R¹ sind H-Atome.

In einer weiteren bevorzugten Ausführungsform der Erfindung weisen alle asymmetrischen Zentren (*) der allgemeinen Verbindung I die gleiche Konfiguration auf.

In einer weiteren bevorzugten Ausführungsform der Erfindung weisen alle asymmetrischen Zentren (*) der allgemeinen Verbindung I die S-Konfiguration auf.

In einer weiteren bevorzugten Ausführungsform der Erfindung weisen alle asymmetrischen Zentren (*) der allgemeinen Verbindung I die R-Konfiguration auf.

Ferner werden erfindungsgemäß Zusammensetzungen offenbart, die eine oder mehrere erfindungsgemäße Verbindungen gegebenenfalls in Kombination mit üblichen Adjuvantien enthalten.

Die Synthese der Verbindungen der allgemeinen Formel I erfolgt vorzugsweise aus enantiomerenreinen Monomeren. Während der Synthese der Verbindungen der allgemeinen Formel I können einzelne asymmetrische Zentren aufgrund der chemischen Synthesebedingungen zu einem geringen Prozentsatz ihre vorher definierte Konfiguration ändern. Der größte Prozentsatz, der während der Synthese gebildeten Verbindungen der allgemeinen Formel I, ist aber stereoisomerenrein. Auch diese Zusammensetzungen sind in der Lage, die erfindungsgemäße Aufgabe zu erfüllen.

Eine Verbindung der allgemeinen Formel I kann über die Reste K und L als Linker mit einer zweiten Verbindung der allgemeinen Formel I verbunden sein wobei die Reste wie oben definert sind. Die Konfiguration an den asymmetrischen Zentren der einen Verbindung der allgemeinen Formel I ist dabei unabhängig von der Konfiguration an den asymmetrischen Zentren der über den Linker verbundenen zweiten Verbindung der allgemeinen Formel **I**. So können beispielweise alle asymmetrischen Zentren der einen Verbindung der allgemeinen Formel I die R-Konfiguration aufweisen und alle asymmetrischen Zentren der zweiten, verbundenen Verbindung der allgemeinen Formel **I** die S-Konfiguration aufweisen. Es können auch beispielweise alle asymmetrischen Zentren der einen Verbindung der allgemeinen Formel **I** die R-Konfiguration aufweisen und alle asymmetrischen Zentren der zweiten, verbundenen Verbindung der allgemeinen Formel **I** die R-Konfiguration aufweisen.

Der Linker dient inbesondere dazu, den Abstand zwischen zwei Verbindungen der allgemeinen Formel I so einzustellen, dass zwischen den beiden Verbindungen der allgemeinen Formel I mit Linker und einzelsträngiger RNA oder DNA bzw. doppelsträngiger DNA eine gegenseitige Interaktion über die jeweiligen Nukleobasen erfolgen kann.

Als Linker eignen sich alle bekannten und alle für diesen Zweck eingesetzten bzw. einsetzbaren Linkermoleküle. Beispielsweise kann ein solcher Linker eine gegebenenfalls substituierte Alkylkette, ein Peptid, ein Oligonukleotid, oder ein Oligomer, das aus mindestens drei 8-Amino-3,6-dioxaoctansäure-Einheiten (eg1-Einheiten) aufgebaut ist, sein.

Die Anzahl und die Reihenfolge der Reste R¹, die mit einer Phosphonsäureester- bzw. Phosphonsäure-Funktion substituiert sind, kann erfindungsgemäß frei gewählt werden. So kann beispielsweise jeder, jeder zweite, jeder dritte, jeder vierte, jeder fünfte, jeder sechste, jeder siebte, jeder achte, jeder neunte oder jeder zehnte Rest R¹ mit einer Phosphonsäureester-bzw. Phosphonsäure-Funktion substituiert sein. Die Substitutionen mit den Phosphonsäureester- bzw. Phosphonsäure-Funktionen können regelmäßig sein oder an beliebigen Positionen vorliegen.

Ferner können auch mehrere Reste R¹ aufeinander folgend mit einer Phosphonsäureester- bzw. Phosphonsäure-Funktion substituiert sein (benachbarte Anordnung). Dabei können in der Verbindung der allgemeinen Formel I auch mehrere dieser benachbarten Anordnungen enthalten sein.

Es können aber auch beispielsweise nur einzelne Reste R¹ an beliebigen Positionen mit einer Phosphonsäureester- bzw. die Phosphonsäure-Funktion substituiert sein.

Die Positionen mit den einzelnen, aufeinander folgenden Reste R¹, die mit einer Phosphonsäureester- bzw. die Phosphonsäure-Funktion substituiert sind, können beliebig sein.

In EP 1157031 werden Verbindungen beschrieben, die mit Phosphonsäureester- oder Phosphonsäure-Funktionen substituiert sind und dadurch eine gute Zellgängigkeit aufweisen.

Im Gegensatz zu den in EP 1157031 beschriebenen Verbindungen sind die hier beschriebenen, erfindungsgemäßen Verbindungen zusätzlich mit mindestens einem Monohydroxy-mononitro-phenyl-Rest oder Monohydroxy-dinitro-phenyl-Rest substituiert.

Bei diesen erfindungsgemäßen Verbindungen haben die Erfinder eine überraschende, selektive Lokalisierung sowohl an als auch in Mitochondrien innerhalb von lebenden Zellen festgestellt. Die erfindungsgemäßen Verbindungen können nach ihrer selektiven Lokalisierung sowohl an als auch in Mitochondrien ihre Wirksamkeit durch einen überraschend starken Antisense- oder antigenomischen Effekt innerhalb der Mitochondrien entfalten.

Zur Feststellung der Lokalisierung sowohl an als auch in Mitochondrien wurden erfindungsgemäße Verbindungen mit dem Fluoreszenz-Farbstoff Biotin gelabelt, um diese in Zellgängigkeitsexperimenten mit einem konfokalen Mikroskop anhand der grünen Fluoreszenz innerhalb der Zellen detektieren zu können. Zur Anfärbung der Mitochondrien wurde kommerziell erhältlicher "MitoTracker" verwendet, mit dessen Hilfe die Mitochondrien mit dem konfokalen Mikroskop anhand der roten Fluoreszenz zu erkennen sind. Gleichzeitig wurde der Zellkern durch "DAPI"-Anfärbung anhand seiner blauen Fluoreszenz identifiziert. Die Zellen wurden mit einer 10µM Lösung der erfindungsgemäßen und Biotin-gelabelten Verbindungen 24 Stunden inkubiert und danach mit dem konfokalen Mikroskop analysiert. Dabei wurden verschiedene Linescans durch die Zellen gemessen.

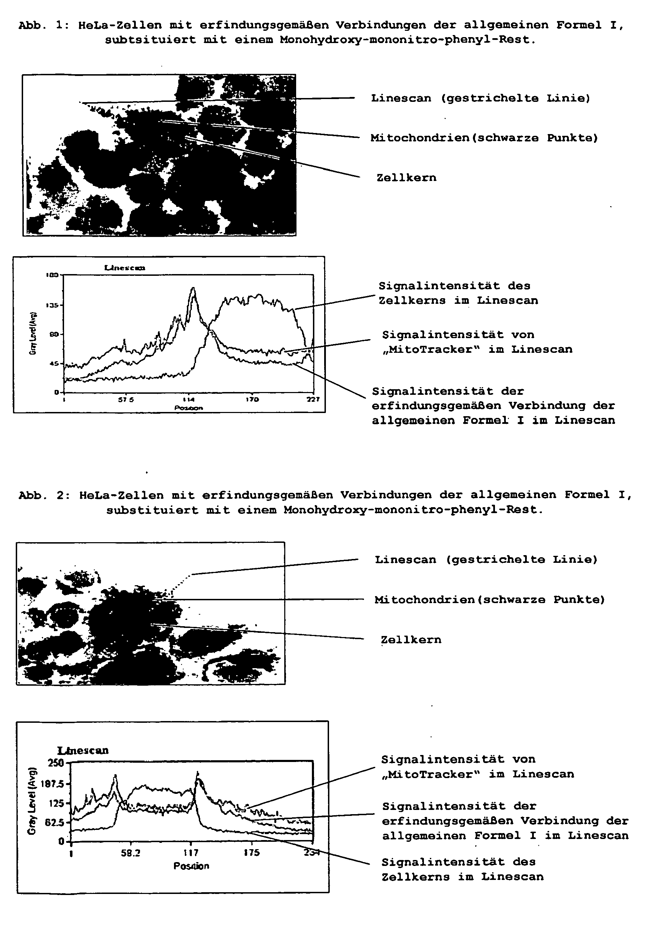

Die Linescan-Analysen in den Abbildungen 1-4 durch die Hela-Zellen bzw. 143B parentalen Zellen zeigen die Signalintensitäten der Verbindungen der allgemeinen Formel I, der Mitochondrien und des Zellkerns.

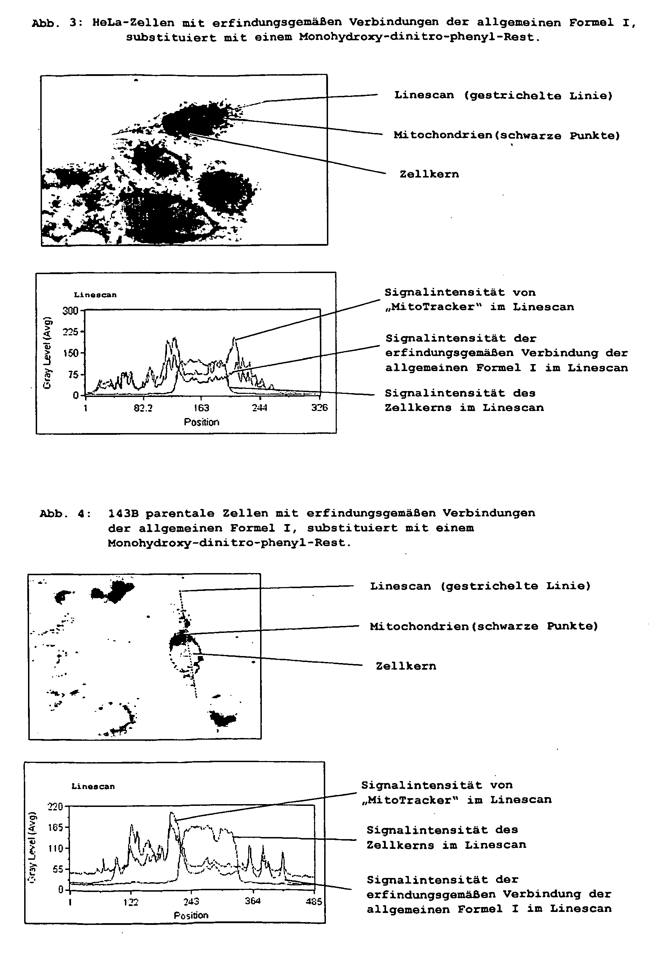

An den parallelen Signalintensitäten der erfindungsgemäßen Verbindungen der allgemeinen Formel I mit einem Monohydroxy-mononitro-phenyl-Rest (Abb. 1-2) bzw. Monohydroxy-dinitro-phenyl-Rest (Abb. 3-4) und der Mitochondrien ist die selektive Lokalisierung der Verbindungen der allgemeinen Formel I sowohl an als auch in den Mitochondrien deutlich zu erkennen. Im Zellkern dagegen sind keine erfindungsgemäßen Verbindungen zu erkennen. Die erfindungsgemäßen Verbindungen zeigen hierbei eine vergleichbare Selektivität in Bezug auf die Lokalisierung sowohl an als auch in Mitochondrien wie das kommerziell erhältliche Mitochondrien-Anfärbe-Reagenz "MitoTracker".

Die erfindungsgemäßen Verbindungen zeigen einen ebenfalls überraschend starken Antisense- und antigenomischen Effekt innerhalb der Mitochondrien. Eine gegen die Expression des mitochondrialen Proteins COX1 gerichtete erfindungsgemäße Verbindung reduziert in HeLa-Zellen bei einer Konzentration von 10µM den Protein-Level von COX1 nach 3 Tagen auf 71% und nach 9 Tagen auf 20% im Vergleich zu unbehandelten HeLa-Zellen. Neben einem zeitabhängigen Effekt ist auch ein konzentrationsabhängiger Effekt beobachtbar. So wird beispielsweise nach 9 Tagen der Protein-Level von Cox1 bei einer Konzentration von 2,5µM auf 55% und bei 500nM noch auf 80% gesenkt.

Auch die Anzahl der Kopien an mitochondrialer DNA in den HeLa-Zellen wird durch die Behandlung mit den erfindungsgemäßen Verbindungen (mit Anti-COX1-Sequenz) ebenfalls zeit- und konzentrationsabhängig reduziert.

Während bei einer Konzentration von 10µM nach 3 Tagen noch kein Effekt festgestellt werden kann, ist nach 6 Tagen eine Reduktion der mtDNA auf 81% und nach 9 Tagen auf 62% beobachtbar. Diese Werte stehen im Vergleich zu unbehandelten HeLa-Zellen bzw. im Vergleich zu HeLa-Zellen, die mit einer erfindungsgemäßen Verbindung behandelt wurden, die keine komplementäre Sequenz zur mtDNA besitzt (Negativ Kontrolle).

Bei den HeLa-Zellen, die mit den erfindungsgemäßen Verbindungen (mit Anti-COX1-Sequenz) mit verschiedenen Konzentrationen behandelt wurden, sind beispielsweise die mtDNA-Kopien nach 9 Tagen bei 10µM auf 62%, bei 2,5µM auf 82% und bei 500nM auf 83% reduziert.

Die erfindungsgemäßen Verbindungen sind damit den bekannten (A. Muratovska, R. N. Lightowlers, R. W. Taylor, D. M. Turnbull, R. A. J. Smith, J. A. Wilce, S. W. Martin, M. P. Murphy, Nucleic Acid Research, 2001, Vol. 29, No. 9, 1852-1863), mit einem Triphenyl-phosphonium-Rest gekoppelten, Peptidnukleinsäuren deutlich überlegen. Die dort beschriebenen Moleküle zeigen nur in zellfreien Systemen eine Bindung an mitochondriale DNA. Darüber hinaus sind diese Moleküle zwar in der Lage, die äußere Zellmembran einer Zelle zu durchdringen und sich an Mitochondrien anzulagern, zeigen aber dann in den Mitochondrien innerhalb von Zellen keine Wirksamkeit, wie zum Beispiel einen Antisense- oder antigenomischen Effekt.

Verbindungen der allgemeinen Formel I, die keinen Monohydroxy-mononitro-phenyl-Rest oder Monohydroxy-dinitro-phenyl-Rest an den Substituenten K, L oder R¹ tragen, verteilen sich entweder gleichmäßig innerhalb von Zellen oder lagern sich in anderen Zellkompartimenten als den Mitochondrien an. Überraschenderweise führt allein die Substitution der Verbindungen der allgemeinen Formel I mit einem Monohydroxy-mononitro-phenyl-Rest oder Monohydroxy-dinitro-phenyl-Rest zu einer selektiven Lokalisierung dieser mitochondrialen Wirkstoffe sowohl an als auch in Mitochondrien.

Verbindungen der vorliegenden Erfindung, die durch die kovalente Kopplung mit einem Monohydroxy-mononitro-phenyl-Rest oder Monohydroxy-dinitro-phenyl-Rest selektiv sowohl an als auch in Mitochondrien innerhalb von Zellen lokalisiert werden, um anschließend ihre Wirksamkeit an oder in Mitochondrien entfalten zu können, können mit oder ohne Hilfe von Transfektionsreagenzien bei einer extrazellulären Konzentration von unter 50 µM durch die äußere Zellmembran in das Zellinnere gelangen.

Die erfindungsgemäßen Verbindungen sind dadurch zur Behandlung von mitochondrialen Krankheiten sowie zu diagnostischen Zwecken im Zusammenhang mit Mitochondrien geeignet. Dies sind beispielsweise Erbkrankheiten, Krebs, Parkinson oder Diabetes. Erfindungsgemäße Verbindungen können auch als anti-aging-Wirkstoffe eingesetzt werden.

Auch die Verwendung der erfindungsgemäßen Verbindungen zur Herstellung von Arzneimitteln zur Vorbeugung und/oder Behandlung von Erkrankungen ist Gegenstand der vorliegenden Erfindung. Im Allgemeinen werden erfindungsgemäße Verbindungen unter Anwendung der bekannten und akzeptablen Modi, entweder einzeln oder in Kombination mit einem beliebigen anderen therapeutischen Mittel verabreicht. Die Verabreichung kann z.B. auf einem der folgenden Wege erfolgen: oral, z.B. als Dragees, überzogene Tabletten, Pillen, Halbfeststoffe, weiche oder harte Kapseln, Lösungen, Emulsionen oder Suspensionen; parenteral, z.B. als injizierbare Lösung; rektal als Suppositorien; durch Inhalation, z.B. als Pulverformulierung oder Spray, transdermal oder intranasal. Zur Herstellung solcher Tabletten, Pillen, Halbfeststoffe, überzogenen Tabletten, Dragees und harten Gelatinekapseln kann das therapeutisch verwendbare Produkt mit pharmakologisch inerten, anorganischen oder organischen Arzneimittelträgersubstanzen vermischt werden, z.B. mit Lactose, Sucrose, Glucose, Gelatine, Malz, Silicagel, Stärke oder Derivaten derselben, Talkum, Stearinsäure oder ihren Salzen, Trockenmagermilch und dgl. Zur Herstellung von weichen Kapseln kann man Arzneimittelträgerstoffe wie z.B. pflanzliche Öle, Petroleum, tierische oder synthetische Öle, Wachse, Fette, Polyole einsetzen. Zur Herstellung von flüssigen Lösungen und Sirups kann man Arzneimittelträgerstoffe wie z.B. Wasser, Alkohole, wässrige Salzlösung, wässrige Dextrose, Polyole, Glycerin, pflanzliche Öle, Petroleum, tierische oder synthetische Öle verwenden. Für Suppositorien kann man Arzneimittelträgerstoffe wie z.B. pflanzliche Öle, Petroleum, tierische oder synthetische Öle, Wachse, Fette und Polyole verwenden. Für Aerosol-Formulierungen kann man komprimierte Gase, die für diesen Zweck geeignet sind, wie z.B. Sauerstoff, Stickstoff, Fluorchlorkohlenwasserstoffe, Fluorkohlenwasserstoffe, Chlorkohlenwasserstoffe und Kohlendioxid einsetzen. Die pharmazeutisch verwendbaren Mittel können auch Zusatzstoffe zur Konservierung, Stabilisierung, Emulgatoren, Süßstoffe, Aromastoffe, Salze zur Veränderung des osmotischen Drucks, Puffer, Umhüllungszusatzstoffe und Antioxidantien enthalten.

Die erfindungsgemäßen Verbindungen der allgemeinen Formel I lassen sich beispielsweise mittels in der Literatur beschriebenen Verfahren durch Umsetzung von Verbindungen der allgemeinen Formel **II** in an sich bekannter Weise (z.B. L. Christensen, R. Fitzpatrick, B. Gildea, K.H. Petersen, H.F. Hansen, T. Koch, M. Egholm, O. Buchardt, P.E. Nielsen, J. Coull, R.H. Berg, J.Pept.Sci. 3, 1995, 175-183. T. Koch, H.F. Hansen, P. Andersen, T. Larsen, H.G. Batz, K. Otteson, H. Örum, J.Pept.Res. 49, 1997, 80-88. F. Bergmann, W. Bannwarth, S. Tam, Tetrahedron Lett. 36, 1995, 6823-6826) herstellen.

Die Einführung von Monohydroxy-mononitro-phenyl-Resten oder Monohydroxy-dinitro-phenyl-Resten als Substituent in den erfindungsgemäßen Verbindungen kann beispielsweise über die Kopplung der Verbindungen der allgemeinen Formel **III** oder **IV** an eine Aminfunktion in den Resten K, L oder R¹ erfolgen.

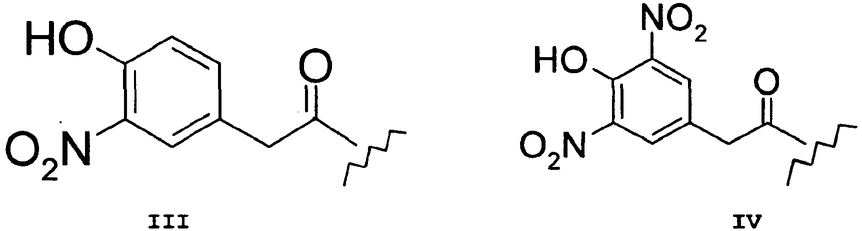

Im Folgenden wird die Verbindung der allgemeinen Formel III als MNPA ("MonoNitro-hydroxy-Phenyl-Acetät") die Verbindung der allgemeinen Formel IV als DNPA ("DiNitro-hydroxy-Phenyl-Acetat") abgekürzt.

In den Verbindungen der allgemeinen Formel **II** 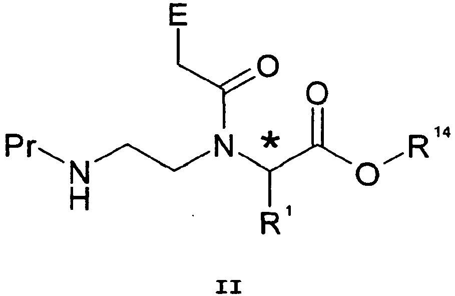 ist der Rest R¹⁴ z.B. ein H-Atom oder ein Allyl-, Benzyl-, Ethyl-, Methyl-Rest oder ein lösliches oder unlösliches Polymer.

Pr ist ein H-Atom oder eine abspaltbare Aminschutzgruppe. Die Aminschutzgruppe muß in Gegenwart der Nukleobasen-Schutzgruppen selektiv abspaltbar sein. Vorzugsweise ist Pr ein H-Atom, eine Oxocarbamat- oder eine Thiocarbamat-Schutzgruppe, am stärksten bevorzugt ist Pr ein H-Atom oder eine Fmoc-, Boc-, Cbz-, Mmt- oder eine Bhoc-Schutzgruppe.

E und der Rest R¹ sind wie oben definiert.

Das asymmetrische Zentrum (*), an das der Rest R¹ bindet, kann die R- oder S-Konfiguration aufweisen.

Die Verbindungen der allgemeinen Formel **II** können zum Beispiel nach folgendem Verfahren hergestellt werden.

Herstellung der Verbindungen der allgemeinen Formel **II** mit R-Konfiguration am asymmetrischen Zentrum:

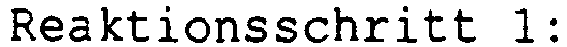

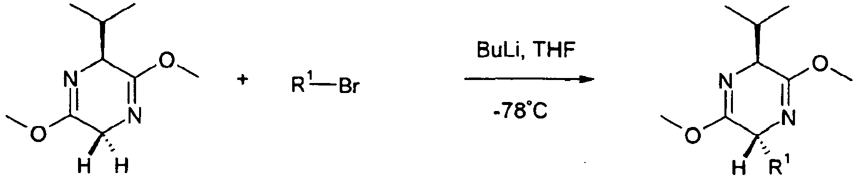

Ausgehend von der S-Konfiguration des Pyrazin-Edukts kann die Durchführung beispielsweise wie in der Literatur beschrieben (U. Schöllkopf, U. Busse, R. Lonsky, R. Hinrichs, Liebigs Ann. Chem. 1986, 2150 -2163; A. Schick, T. Kolter, A. Giannis, K. Sandhoff, Tetrahedron 51, 1995, 11207-11218) erfolgen.

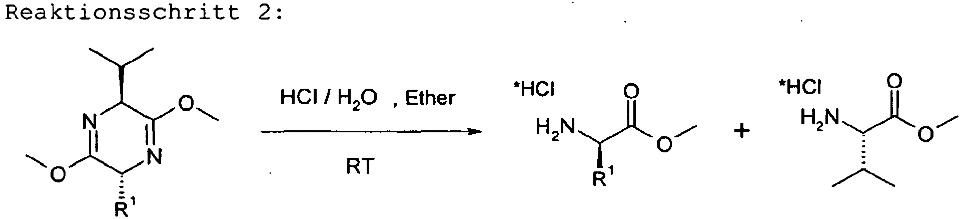

Die Durchführung kann beispielsweise wie in der Literatur beschrieben (U. Schöllkopf, U. Busse, R. Lonsky, R. Hinrichs, Liebigs Ann. Chem. 1986, 2150 -2163) erfolgen.

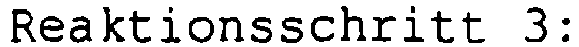

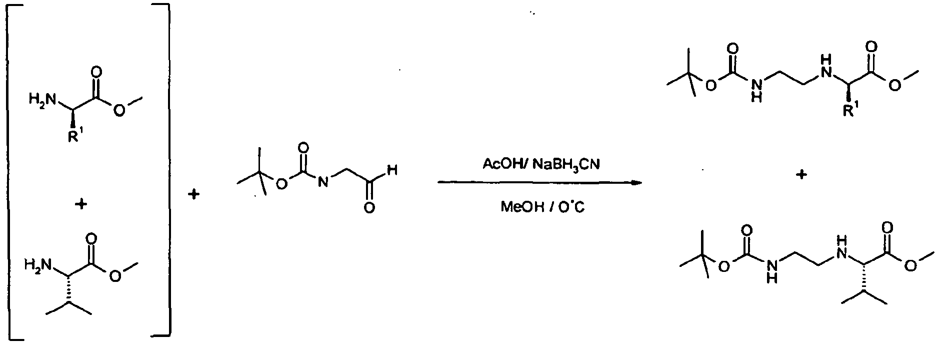

Das Produktgemisch aus Reaktionsschritt 2 kann, nach Freisetzung der Amine aus ihren Hydrochloriden durch eine Base (z.B. NaHCO₃, NH₃), in die Folgereaktion eingesetzt werden. Diese, eine reduktive Aminierung, kann wie in der Literatur beschrieben (G. Haaima, A. Lohse, O. Buchardt, P.E. Nielsen, Angew. Chem. Int. Ed. Engl. 35, 1996, No 17, 1939-1942) durchgeführt werden.

Statt Natriumcyanoborhydrid können auch andere Reduktionsmittel wie z. B. Wasserstoff und ein Katalysator (z. B. Pd/C) verwendet werden. Die Reaktionsprodukte werden chromatographisch aufgetrennt.

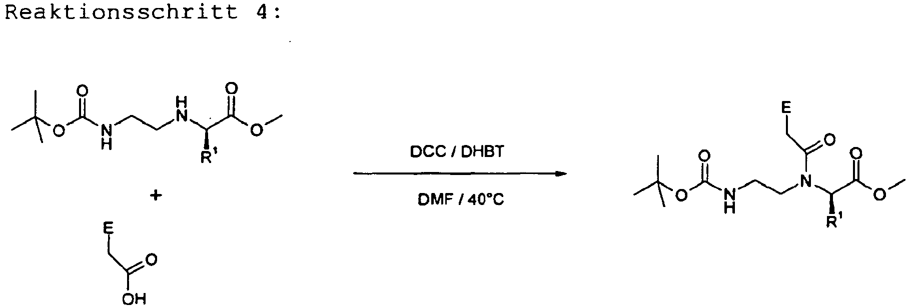

Die Durchführung kann beispielsweise wie in der Literatur beschrieben (G. Haaima, A. Lohse, O. Buchardt, P.E. Nielsen, Angew. Chem. Int. Ed. Engl. 35, 1996, No 17, 1939-1942) erfolgen. Dabei können auch andere Kupplungsreagentien an Stelle von DCC /DHBT eingesetzt werden.

Die Herstellung der Verbindung E-CH₂-COOH (zum Beispiel C(PG)-CH₂-COOH, A(PG)-CH₂-COOH, G(PG)-CH₂-COOH oder T-CH₂-COOH, J(PG)-CH₂-COOH wobei A = Adeninyl, C = Cytosinyl, G = Guaninyl, T = Thyminyl, J = Pseudoisocytosinyl PG = Schutzgruppe wie beispielsweise Benzyloxycarbonyl (Z), Benzyl (Bzl), Acetyl(Ac) oder Anisoyl (An)) kann beispielsweise wie in der Literatur beschrieben (S.A. Thomson, J.A. Josey, R.Cadilla, M.D. Gaul, F.C. Hassmann, M.J. Lazzio, A.J. Pipe, K.L. Reed, D.J. Ricca, R.W. Wiether, S.A. Noble, Tetrahedron 51, 1995, 6179-6194) erfolgen. Weitere mögliche Schutzgruppen sind ebenfalls in der Literatur beschrieben (G. Breitpohl, D.W. Will, A. Peymann, E. Uhlmann, Tetrahedron 53, 1997, 14671-14686; T. Kofoed, H.F. Hansen, H. Orum, T. Koch, J. Peptide Sci., 7, 2001, 402-412)

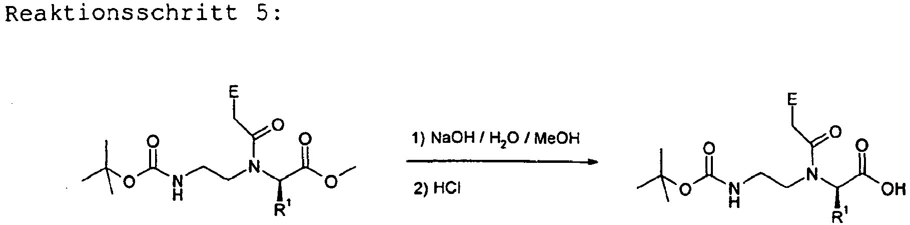

Die Durchführung kann beispielsweise wie in der Literatur beschrieben (G. Haaima, A. Lohse, O. Buchardt, P.E. Nielsen, Angew. Chem. Int. Ed. Engl. 35, 1996, No 17, 1939-1942) erfolgen.

Zur einfacheren Beschreibung der Verbindungen der allgemeinen Formel II, die als Produkte in dem Reaktionsschritt 5 entstehen, werden folgende Abkürzungen verwendet:
Wird beispielsweise A(PG)-CH₂-COOH im Reaktionsschritt 5 eingesetzt, so erhält man die entsprechende Verbindung der allgemeinen Formel **II,** die ein asymmetrisches Zentrum aufweist. Diese Verbindung wird hier im Allgemeinen als A^{R}(PG) abgekürzt. Dabei steht A für die Nukleobase in der Verbindung der allgemeinen Formel **II** mit einem asymmetrischen Zentrum, das hochgestellte R steht für die R-Konfiguration der Verbindung und das PG steht für die Schutzgruppe an der Nukleobase.

Wird beispielsweise Phenylessigsäure im Reaktionsschritt 5 eingesetzt, so erhält man eine Verbindung der allgemeinen Formel **II** mit einem asymmetrischen Zentrum, die als P^{R} abgekürzt wird.

Die entsprechenden Verbindungen der allgemeinen Formel **II** ohne asymmetrisches Zentrum (R¹ = H) werden analog zu den Verbindungen der allgemeinen Formel **II** mit einem asymmetrischen Zentrum abgekürzt, mit dem Unterschied, dass anstatt des Großbuchstabens für die Nukleobase und des hochgestellten Buchstabens für die Konfiguration (z.B. A^{R}) der entsprechende Kleinbuchstabe a verwendet wird. Beispielsweise wird eine Verbindung der allgemeinen Formel **II** ohne asymmetrisches Zentrum mit PGgeschütztem C als Nukleobase als c(PG) abgekürzt.

Für die Herstellung der Verbindungen der allgemeinen Formel **II** mit S-Konfiguration am asymmetrischen Zentrum wird im Reaktionsschritt 1 das Pyrazin-Edukt mit der R-Konfiguration eingesetzt und die Reaktionsschritte 1 bis 5 analog durchgeführt. Man erhält dann beispielsweise eine Verbindung der allgemeinen Formel **II** mit der Abkürzung A^{S}(PG).

Die erfindungsgemäßen Verbindungen lassen sich beispielsweise mittels Festphasensynthese durch Umsetzung von Verbindungen der allgemeinen Formel **II** in an sich bekannter Weise herstellen. Nach der Festphasensynthese werden die Schutzgruppen an den Nukleobasen abgespalten, so dass man Verbindungen der allgemeinen Formel **I** erhält, die wie folgt abgekürzt werden:
Beispielsweise wird eine erfindungsgemäße Verbindung, die nur aus Verbindungen der allgemeinen Formel **II** mit einem asymmetrischen Zentrum mit R-Konfiguration hergestellt und welche im letzten Schritt mit MNPA-OH gekuppelt und danach als primäres Amid vom Harz abgespalten wurde, abgekürzt als MNPA-A^{R}C^{R}G^{R}G^{R}T^{R}C^{R}G^{R}G^{R}C^{R}G^{R}A^{R}A^{R}C^{R}A^{R}T^{R}-NH₂.
Beispielsweise wird eine erfindungsgemäße Verbindung, die aus Verbindungen der allgemeinen Formel **II** mit einem asymmetrischen Zentrum mit R-Konfiguration und Verbindungen der allgemeinen Formel **II** ohne asymmetrisches Zentrum hergestellt und welche im letzten Schritt mit MNPA-OH gekuppelt und danach als primäres Amid vom Harz abgespalten wurde, abgekürzt als MNPA-A^{R}cG^{R}gT^{R}cG^{R}gC^{R}gA^{R}aC^{R}aT^{R}-NH₂.
Beispielsweise wird eine erfindungsgemäße Verbindung, die nur aus Verbindungen der allgemeinen Formel **II** mit einem asymmetrischen Zentrum mit S-Konfiguration hergestellt und welche im letzten Schritt mit DNPA-OH gekuppelt und danach als primäres Amid vom Harz abgespalten wurde, abgekürzt als DNPA-A^{S}C^{S}G^{S}G^{S}T^{S}C^{S}G^{S}G^{S}C^{S}G^{S}A^{S}A^{S}C^{S}A^{S}T^{S}-NH₂.
Beispielsweise wird eine erfindungsgemäße Verbindung, die nur aus Verbindungen der allgemeinen Formel **II** mit einem asymmetrischen Zentrum mit S-Konfiguration und Verbindungen der allgemeinen Formel **II** ohne asymmetrisches Zentrum hergestellt und welche im letzten Schritt mit DNPA gekuppelt und danach als primäres Amid vom Harz abgespalten wurde, abgekürzt als DNPA-A^{S}cG^{S}gT^{S}cG^{S}gC^{S}gA^{S}aC^{S}aT^{S}-NH₂.
Beispielsweise wird eine erfindungsgemäße Verbindung, die an einem Boc-Gly-PAM-MBHA-Harz nur aus Verbindungen der allgemeinen Formel **II** mit einem asymmetrischen Zentrum mit R-Konfiguration und Verbindungen der allgemeinen Formel **II** ohne asymmetrisches Zentrum hergestellt und welche im letzten Schritt mit DNPA gekuppelt und danach als primäres Amid vom Harz abgespalten wurde, abgekürzt als DNPA-tG^{R}cC^{R}tA^{R}ggactc^{R}cA^{R}gC^{R}-Gly-NH₂. Beispielsweise wird eine erfindungsgemäße Verbindung, die an einem Boc-Gly-PAM-MBHA-Harz nur aus Verbindungen der allgemeinen Formel **II** mit einem asymmetrischen Zentrum mit R-Konfiguration und Verbindungen der allgemeinen Formel **II** ohne asymmetrisches Zentrum, Glycin sowie zwei Aminosäuren wie 4-(Diethoxy-phosphoryl)-2-(tert.butoxycarbonylamino)-buttersäure (Boc-DEPABS) hergestellt und welche im letzten Schritt mit DNPA gekuppelt und danach als primäres Amid vom Harz abgespalten wurde, abgekürzt als DNPA- (DEPABS)₂-Gly-tG^{R}cC^{R}tA^{R}ggactc^{R}CA^{R}gC^{R}-Gly-NH₂. Beispielsweise wird eine erfindungsgemäße Verbindung, die an einem Boc-Gly-PAM-MBHA-Harz aus Verbindungen der allgemeinen Formel **II** mit einem asymmetrischen Zentrum mit R-Konfiguration und Verbindungen der allgemeinen Formel II ohne asymmetrisches Zentrum, dem Chelator 1,4,7,10-Tetraazacyclododecane-1,4,7,10-tetraessigsäure-tri-tert.butylester (DOTA) hergestellt und welche im letzten Schritt mit DNPA gekuppelt und danach als primäres Amid vom Harz abgespalten wurde, abgekürzt als DNPA-DOTA-gG^{R}cT^{R}cG^{R}aA^{R}tA^{R}aG^{R}gA^{R}gG^{R}-Gly-NH₂.

### Beispiele

### Beispiel 1: Herstellung von (2R,5S)-2-(2-(Diethoxy-phosphoryl)ethyl)-2,5-dihydro-3,6-dimethoxy-5-isopropyl-pyrazin

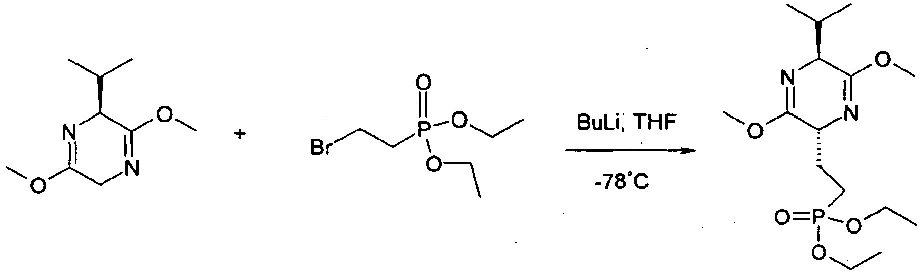

0,52 mol (S)-2,5-Dihydro-3,6-dimethoxy-2-isopropylpyrazin werden in 400 ml absolutem THF unter Argon gelöst und auf -78°C gekühlt. Unter Rühren werden 200 ml einer 2,7 M Butyllithium-Lösung (in Heptan) (0,54 mol) langsam zugetropft. Anschließend tropft man eine Lösung von 0,52 mol Diethyl-(2-bromethyl)-phosphonat in 300 ml absolutiertem THF langsam unter Rühren zu und rührt weitere 3 h bei -78°C. Dann werden 11,7 ml (ca. 0,2 mol) wasserfreie Essigsäure langsam zugegeben. Man lässt die Reaktionsmischung dann langsam auf Raumtemperatur erwärmen. Das Lösemittel wird entfernt, der Rückstand in 600 ml Diethylether aufgenommen und mit 200 ml Wasser gewaschen. Die wässrige Phase wird noch 3-mal mit je 100 ml Diethylether extrahiert. Die vereinigten Etherphasen werden über MgSO₄ getrocknet, filtriert und das Lösemittel wird im Vakuum entfernt. Der Rückstand wird in Et₂O/Hexan (1/10) aufgenommen und über ein Kieselgelbett filtriert. Danach wird nicht umgesetztes Edukt mit Et₂O /Hexan (1/5) eluiert. Abschließend wird dann das Produkt mit Essigester eluiert.
Ausbeute: ca. 70 %, Gelbe Flüssigkeit
**¹H-NMR** (CDCl₃): 0.71, 1.04 (d, 6H, CH(C*H*₃)₂), 1.33 (t, 6H, P(O)(OCH₂C*H*₃)₂), 1.68-2.25 (m, 4H, CHC*H*₂C*H*₂P), 3. 65, 3. 67 (s, 6H, OC*H*₃), 4.02 (m, 1H), 4.10-4.20 (m, 4H, P(O)(OC*H*₂CH₃)₂).

### Beispiel 2: Herstellung von (2R,5S)-2-(8-(Dibenzyloxy-phosphoryl)octyl)-2,5-dihydro-3,6-dimethoxy-5-isopropyl-pyrazin

Analog dem Herstellungsverfahren in Beispiel 1 wird ausgehend von (S)-2,5-Dihydro-3,6-dimethoxy-2-isopropylpyrazin und Dibenzyl-(8-bromoctyl)-phosphonat (2R,5S)-2-(8-(Dibenzyloxy-phosphoryl)octyl)-2,5-dihydro-3,6-dimethoxy-5-isopropyl-pyrazin hergestellt.

### Beispiel 3: Herstellung von (2S,5R)-2-(4-(Dicyclohexyloxy-phosphoryl)but-2-enyl)-2,5-dihydro-3,6-dimethoxy-5-isopropyl-pyrazin

Analog dem Herstellungsverfahren in Beispiel 1 wird ausgehend von (R)-2,5-Dihydro-3,6-dimethoxy-2-isopropylpyrazin und Dicyclohexyl-(4-brom-but-2-enyl)-phosphonat (2S,5R)-2-(4-(Dicyclohexyloxy-phosphoryl)but-2-enyl)-2,5-dihydro-3,6-dimethoxy-5-isopropyl-pyrazin hergestellt.

### Beispiel 4: Herstellung von (2R)-2-[2-(tert.Butoxycarbonylamino)ethyl]-amino-4-(diethoxy-phosphoryl)-buttersäuremethylester

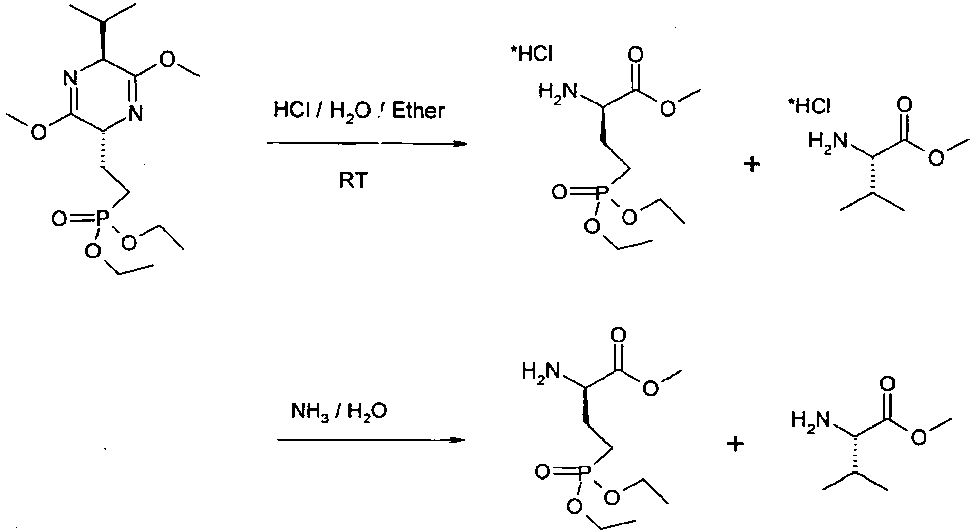

0,38 mol (2R,5S)-2-(2-(Diethoxy-phosphoryl)ethyl)-2,5-dihydro-3,6-dimethoxy-5-isopropyl-pyrazin werden in 400 ml Diethylether gelöst. Zu dieser Lösung werden 1150 ml einer 1N wässrigen HCl-Lösung zugefügt. Nach 60 min ist die Reaktion beendet, und der Ether wird entfernt. Soll das Produkt gelagert werden, wird auch das Wasser komplett im Vakuum entfernt.

Soll das Produkt gleich weiter umgesetzt werden, wird das Wasser etwa zur Hälfte abrotiert, dann wird der pH-Wert des Reaktionsgemisches mit Ammoniaklösung auf 8-9 eingestellt. Die basische Lösung wird 6x mit Dichlormethan extrahiert, wobei jedes Mal der pH-Wert kontrolliert und nötigenfalls korrigiert wird. Die Dichlormethan-Phasen werden vereinigt, mit MgSO₄ getrocknet, und das Lösungsmittel im Vakuum entfernt. Das resultierende gelbe Öl wird sofort in der Folgereaktion, der reduktiven Aminierung eingesetzt.

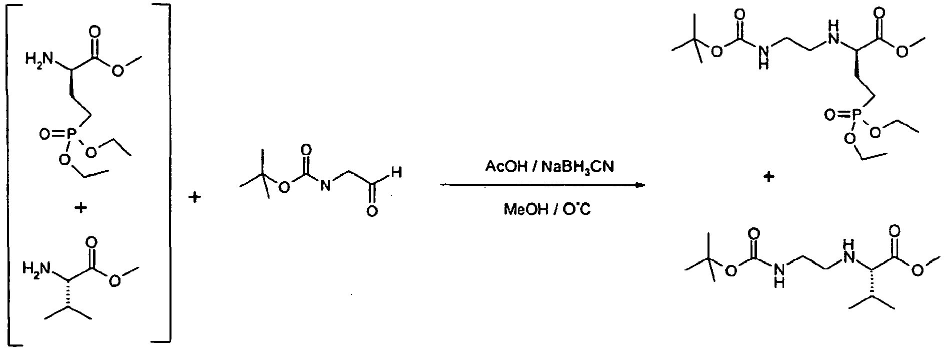

Das gelbe Öl (angenommen wird ein vollständiger Umsatz) wird in 600 ml Methanol aufgenommen und auf 0°C gekühlt. Anschließend werden 0,76 mol N-Boc-Aminoacetaldehyd zugegeben. Nachdem 30 min. bei 0°C gerührt wurde, werden erst 0,90 mol wasserfreie Essigsäure, dann 0,40 mol Natriumcyanoborhydrid zugefügt. Die Reaktionsmischung wird bei 0°C gerührt, bis die Gasentwicklung beendet ist, dann wird das Lösemittel am Rotationsverdampfer entfernt. Der Rückstand wird in Essigsäureethylester aufgenommen (ca. 600 ml) und einmal mit gesättigter NaHCO₃-Lsg. (ca. 200 ml) und einmal mit gesättigter NaCl-Lsg. (ca. 100 ml) gewaschen. Die organische Phase wird mit MgSO₄ getrocknet und filtriert. Anschließend wird das Lösungsmittel im Vakuum entfernt.

Die weitere Aufreinigung erfolgt durch SPE über eine mit Kieselgel gefüllte Glasfritte. Verunreinigungen und unerwünschte Produkte werden zuerst mit Hexan/Essigester 1:1, dann mit reinem Essigester eluiert. Das gewünschte Produkt wird schließlich durch Extraktion mit 10% Methanol in Dichlormethan erhalten.

Nach Entfernen des Lösemittels werden ca. 75% Produkt als gelbes viskoses Öl erhalten.

**¹H-NMR** (CDCl₃): 1.35 (t, 6H, P(O)(OCH₂C*H*₃)₂), 1.47 (s, 9H, C(C*H*₃)₃); 1.8-2.0 (m, 4H, CHC*H*₂C*H*₂P,), 2.5-2.6, 2.75-2.85, 3.0-3.4 (m, 4H, NC*H*₂C*H*₂N), 3.75 (s, 3H, OC*H*₃), 4.0-4.2 (m, 4H, P(O)(OCH₂CH₃)₂).

### Beispiel 5: Herstellung von (2R)-2-[2-(tert.Butoxycarbonylamino)ethyl]-amino-10-(dibenzyloxy-phosphoryl)-decansäuremethylester

Analog dem Herstellungsverfahren in Beispiel 4 wird ausgehend von (2R,5S)-2-(8-(Dibenzyloxy-phosphoryl)octyl)-2,5-dihydro-3,6-dimethoxy-5-isopropyl-pyrazin (2R)-2-[2-(tert.Butoxycarbonylamino)ethyl]-amino-10-(dibenzyloxy-phosphoryl)-decansäuremethylester hergestellt.

### Beispiel 6: Herstellung von (2S)-2-[2-(tert.Butoxycarbonylamino)ethyl]-amino-6-(dicyclohexyloxy-phosphoryl)-hex-4-ensäuremethylester

Analog dem Herstellungsverfahren in Beispiel 4 wird ausgehend von (2S,5R)-2-(4-(Dicyclohexyloxy-phosphoryl)but-2-enyl)-2,5-dihydro-3,6-dimethoxy-5-isopropyl-pyrazin (2S)-2-[2-(tert.Butoxycarbonylamino)ethyl]-amino-6-(dicyclohexyloxy-phosphoryl)-hex-4-ensäuremethylester hergestellt.

### Beispiel 7: Herstellung von (R)-2-([2-{N4-Benzyloxycarbonylcytosin-1yl}-acetyl]-[2-tert.butoxycarbonylamino-ethyl]-amino)-4-(diethoxy-phosphoryl)-buttersäure-methylester

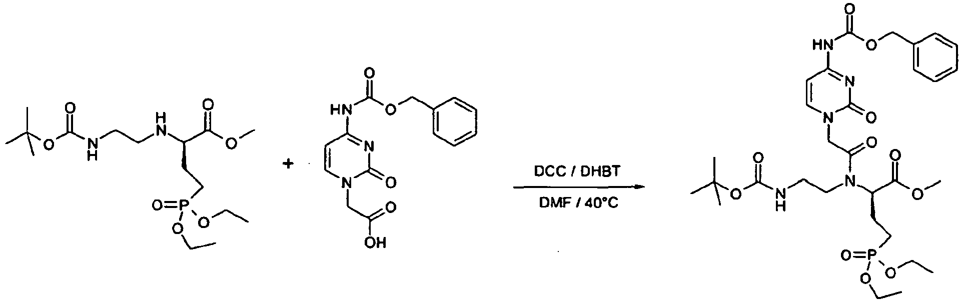

Zu einer gerührten Lösung aus 30,96 mmol 4-N-(Benzyloxycarbonyl)-cytosin-1-yl-essigsäure, und 30,96 mmol 3,4-Dihydro-3-hydroxy-4-oxo-1,2,3-benzotriazin (DHBT-OH) in 100 ml absolutem DMF werden 32,51 mmol DCC gegeben, und diese Lösung wird für 1 h bei 40°C gerührt. Anschließend werden 23,84 mmol (2R)-2-[2-(tert.Butoxycarbonylamino)ethyl]-amino-4-(diethoxy-phosphoryl)-buttersäuremethylester gegeben und bei 40° gerührt. Die Reaktion wird mit HPLC verfolgt und ist nach 3 Tagen beendet.

Vom Unlöslichen wird abfiltriert, das Lösemittel wird im Vakuum entfernt. Der Rückstand wird in Dichlormethan aufgenommen und über Nacht in den Kühlschrank gestellt. Dabei fällt weiterer Dicyclohexylharnstoff aus, der abfiltriert wird. Das Filtrat wird 2-3x mit verdünnter NaHCO₃-Lösung (1/3 ges. NaHCO₃-Lsg., 2/3 Wasser), 1-2x mit verdünnter KHSO₄-Lösung (1/3 gesättigter KHSO₄-Lsg., 2/3 Wasser) gewaschen, mit MgSO₄ getrocknet und einrotiert. Die weitere Aufreinigung erfolgt durch Lösen in Essigester und Lagerung über Nacht im Kühlschrank, wonach eventuell weiterer ausgefallener Dicyclohexylharnstoff abfiltriert und das Lösemittel wieder entfernt wird. Das Rohprodukt wird dann in Dichlormethan gelöst (5 mL je 3 g Rohprodukt) und mit Diethylether (25 mL je 3 g Rohprodukt) und Hexan (5 mL je 3 g Rohprodukt) wieder ausgefällt. Das Lösemittel mit Verunreinigungen wird entfernt und das Produkt in Vakuum getrocknet.
Ausbeute: ca. 65% hellgelber Feststoff
**¹H-NMR** (CDCl₃): 1.32 (t, 6H, P (O)(OCH₂C*H*₃)₂); 1.44 (s, 9H, C(C*H*₃)₃); 1.75-2.45 (m, 4H, CHC*H*₂C*H*₂P); 3.2-3.85 (m, 4H, NC*H*₂C*H*₂N); 3.73 (s, 3H, OC*H*₃); 4.07 (m, 4H, P(O)(OC*H*₂CH₃)2); 4.28 (m, 1H, NC*H*C(O)); 4.42/4.99 (2d, 2H, NC*H*₂C(O)); 5.22 (s, 2H, OC*H*₂Ph); 5.56 (t, br, 1H, C(O)N*H*CH₂); 7.25 (d, 1H, CC*H*=CHN); 7.38 (s, 5H, Ph);7.55 (d, 1H, CCH=C*H*N).

### Beispiel 8: Herstellung von (R)-2-([2-{N4-Benzyloxycarbonylamino-cytosin-lyl}-acetyl]-[2-tert.butoxycarbonylamino-ethyl]-amino)-4-(diethoxy-phosphoryl)-buttersäure

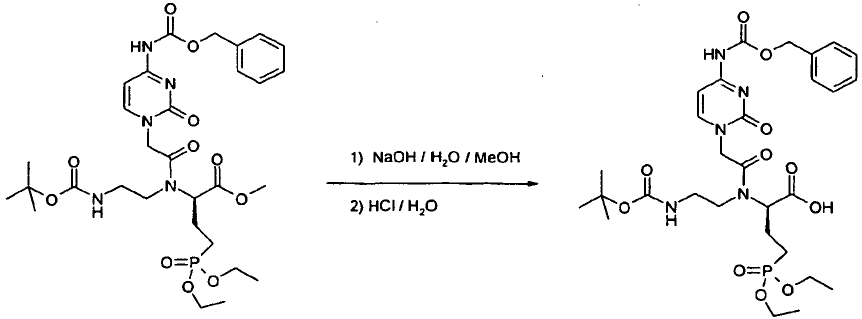

19,1 mmol (R)-2-([2-{N4-Benzyloxycarbonylcytosin-1-yl}-acetyl]-[2-tert.butoxycarbonylamino-ethyl]-amino)-9-(diethoxy-phosphoryl)-buttersäure-methylester wird in 80 ml THF / Wasser (2/3) gelöst und auf 0°C gekühlt. Dazu werden 48 ml einer 1M Lithiumhydroxid-Lösung getropft (pH ∼ 9). Der Fortschritt der Reaktion wird mit DC (10% Methanol in Dichlormethan) verfolgt. Nach Abschluss der Reaktion wird die Reaktionslösung mit 130 ml Wasser / NaCl-Lösung verdünnt und einmal mit Dichlormethan (200 ml) extrahiert. Die wässrige Phase wird mit 2M KHSO₄-Lösung auf pH 2-3 eingestellt und mehrfach mit Dichlormethan extrahiert. Dabei wird immer wieder der pH-Wert kontrolliert und nötigenfalls nachkorrigiert. Die vereinigten organischen Phasen werden über MgSO₄ getrocknet, und das Lösungsmittel im Vakuum entfernt. Wenn notwendig, kann das Rohprodukt aus Dichlormethan mit Diethylether umgefällt werden. Schließlich wird das Produkt am Lyophylisator getrocknet.
Ausbeute: ca. 80 % weißgelber Feststoff
**¹H-NMR** (DMSO-d₆): 1.21 (t, 6H, P (O)(OCH₂C*H*₃)₂); 1.39 (s, 9H, C(C*H*₃)₃); 1.70-2.30 (m, 4H, CHC*H*₂C*H*₂P); 2.90-3.60 (m, 4H, NC*H*₂C*H*₂N); 3.93-4.02 (m, 4H, P(O)(OC*H*₂CH₃)₂); 4.25 (m, 1H, NC*H*C(O)); 4.50-4.83 (m, 2H, NC*H*₂C(O)); 5.19 (s, 2H, OC*H*₂Ph); 6.88 (m, br, 1H, C(O)N*H*CH₂); 7.02 (d, 1H, CC*H*=CHN); 7.31-7.41 (m, 5H, Ph); 7.97 (d, 1H, CCH=C*H*N).

### Beispiel 9: Herstellung von weiteren Verbindungen der allgemeinen Formel II

Durch analoge Synthesen wie in den Beispielen 7 und 8 beschrieben, bei denen neben C(Z)-CH₂-COOH weitere Z-geschützte, Benzyl-geschützte (Bzl), Anisoyl-geschützte (An) bzw. Acetylgeschützte (Ac) und ungeschützte Nukleobasen-Essigsäure-Komponenten A(Z)-CH₂-COOH, A(An)-CH₂-COOH, A(Bzl)-CH₂-COOH, G(Z)-CH₂-COOH, G(Ac)-CH₂-COOH, C(An)-CH₂-COOH, C(Bzl)-CH₂-COOH, J(Z)-CH₂-CCOH, J(Bzl)-CH₂-COOH, J(An)-CH₂-COOH bzw. T-CH₂-COOH (A = Adeninyl, C = Cytosinyl, G = Guaninyl, T = Thyminyl; J = Pseudoisocytosinyl) sowie Phenylessigsäure eingesetzt werden, werden weitere erfindungsgemäße Verbindungen der allgemeinen Formel II hergestellt.
**A^{R}(Z)**:
   **¹H-NMR** (CH₃OH-d₄): 1.20 (t, 6H, P(O)(OCH₂C*H*₃)₂); 1.34 (s, 9H, C(C*H*₃)₃); 1.70-2.30 (m, 4H, CHC*H*₂C*H*₂P); 3.00-3.80 (m, 4H, NC*H*₂C*H*₂N); 3.93-4.02 (m, 4H, P(O)(OC*H*₂CH₃)₂); 4.10 (m, 1H, NC*H*C(O)); 5.18 (s, 2H, OC*H*₂Ph); 5.20-5.40 (m, 2H, NC*H*₂C(O)); 7.15-7.40 (m, 5H, *Ph*); 8.14 (s, 1H, N=CHN); 8.46 (s, 1H, N=CHN).
**A^{R} (Bzl) :**
   **¹H-NMR** (DMSO-d₆): 1.21 (t, 6H, P(O)(OCH₂C*H*₃)₂), 1.40 (s, 9H, C(C*H*₃)₃); 1.70-2.20 (m, 4H, CHC*H*₂C*H*₂P,), 2.90-3.75 (m, 4H, NC*H*₂C*H*₂N); 3.90-4.10 (m, 4H, P(O)(OC*H*₂CH₃)₂); 4.22 (m, 1H, NC*H*C(O)); 5.25-5.45 (m, 2H, NC*H*₂C(O)); 6.96 (m, br, 1H, C(O)N*H*CH₂); 7.50-8.10 (m, 5H, *Ph);* 8.42 (s, 1H, N=CHN); 8.69 (s, 1H, N=CHN).
**A^{R} (An)**:
   **¹H-NMR** (DMSO-d₆): 1.21 (t, 6H, P(O)(OCH₂C*H*₃)₂); 1.41 (s, 9H, C(C*H*₃)₃); 1.70-2.20 (m, 4H, CHC*H*₂C*H*₂P); 2.90-3.750 (m, 4H, NC*H*₂C*H*₂N); 3.86 (s, 3H, OC*H*₃); 3.90-4.10 (m, 4H, P(O)(OC*H*₂CH₃)₂); 4.22 (m, 1H, NC*H*C(O)); 5.25-5.45 (m, 2H, NC*H*₂C(O)); 6.96 (m, br, 1H, C(O)N*H*CH₂); 7.08 (d, 2H, *Ph) ;* 8.05 (d, 2H, *Ph) ;* 8.42 (s, 1H, N=CHN); 8.69 (s, 1H, N=C*H*N).
**J^{R}(Z):**
   **¹H-NMR** (DMSO-d₆): 1.32 (t, 6H, P(O)(OCH₂C*H*₃)₂), 1.42 (s, 9H, C(C*H*₃)₃); 1.60-2.50 (m, 4H, CHC*H*₂C*H*₂P,), 3.10-3.55 (m, 4H, NC*H*₂C*H*₂N); 3.65-3.90 (m, 2H, NC*H*₂C(O)); 4.00-4.15 (m, 4H, P(O)(OC*H*₂CH₃)₂); 4.20 (m, 1H, NC*H*C(O)); 5.24 (s, 2H, OC*H*₂Ph); 6.80 (m, br, 1H, C(O)N*H*CH₂); 7.27 (d, 1H, C=CHN) ; 7.30-7.50 (m, 5H, *Ph*).
**J^{R}(An):**
   **¹H-NMR** (DMSO-d₆): 1.22 (t, 6H, P(O)(OCH₂C*H*₃)₂); 1.38 (s, 9H, C(C*H*₃)₃); 1.65-2.25 (m, 4H, CHC*H*₂C*H*₂P); 2.80-3.70 (m, 4H, NC*H*₂C*H*₂N); 2.80-3.70 (m, 2H, CC*H*₂C(O)); 3.84 (s, 3H, OC*H*₃); 3.90-4.05 (m, 4H, P(O)(OCH₂CH₃)₂); 4.17 (m, 1H, NC*H*C(O)); 6.81 (m, br, 1H, C(O)N*H*CH₂); 7.05 (d, 2H, Ph); 7.70 (s, 1H, NCH=C); 8.07 (d, 2H, *Ph*)*.*
**G^{R}(Z)**:
   **¹H-NMR** (DMSO-d₆): 1.18 (t, 6H, P (O)(OCH₂CH₃)₂), 1.37 (s, 9H, C(C*H*₃)₃); 1.70-2.30 (m, 4H, CHC*H*₂C*H*₂P,), 2.95-3.70 (m, 4H, NC*H*₂C*H*₂N); 3.90-4.10 (m, 4H, P(O)(OCH₂C*H*₃)₂); 4.20 (m, 1H, NC*H*C(O)); 4.85-5.20 (m, 2H, NC*H*₂C(O)); 5.269 (s, 2H, OC*H*₂Ph); 6.95 (m, br, 1H, C(O)N*H*CH₂); 7.30-7.50 (m, 5H, *Ph*); 7.85 (s, 1H, N=C*H*N).
**G^{R}(Ac)**:
   **¹H-NMR** (DMSO-d₆): 1.20 (t, 6H, P(O)(OCH₂C*H*₃)₂); 1.41 (s, 9H, C(C*H*₃)₃); 1.70-2.18 (m, 4H, CHC*H*₂C*H*₂P); 2.20 (s, 3H, C*H*₃C(O)); 2.90-3.60 (m, 4H, NC*H*₂C*H*₂N); 3.90-4.10 (m, 4H, P(O)(OCH₂C*H*₃)₂); 4.22 (m, 1H, NC*H*C(O)); 4.91-5.22 (m, 2H, NCH₂C(O)); 7.00 (m, br, 1H, C(O)N*H*CH₂); 7.88 (s, 1H, N=C*H*-N);.
**C^{R}**(**Bzl**)**:**
   **¹H-NMR** (DMSO-d₆): 1.21 (t, 6H, P (O)(OCH₂C*H*₃)₂); 1.40 (s, 9H, C(C*H*₃)₃); 1.70-2.30 (m, 4H, CHC*H*₂C*H*₂P); 3.20-3.60 (m, 4H, NC*H*₂C*H*₂N); 3.93-4.02 (m, 4H, P(O)(OC*H*₂CH₃)₂); 4.28 (m, 1H, NC*H*C(O)); 4.50-4.83 (m, 2H, NC*H*₂C(O)); 6.90 (m, br, 1H, C(O)N*H*CH₂); 7.33 (d, 1H, CC*H*=CHN); 7.50-7.55 (m, 2H, Ph); 7.62 (d, 1H, CCH=C*H*N); 8.00-8.10 (m, 3H, Ph).
**C^{R}(An) : ¹H-NMR** (DMSO-d₆): 1.22 (t, 6H, P(O)(OCH₂C*H*₃)₂); 1.39 (s, 9H, C(C*H*₃)₃); 1.65-2.10 (m, 4H, CHC*H*₂C*H*₂P); 3.20-3.60 (m, 4H, NC*H*₂C*H*₂N); 3.84 (s, 3H, OC*H*₃); 3.85-4.05 (m, 4H, P(O)(OC*H*₂CH₃)₂); 4.25 (m, 1H, NC*H*C(O)); 4.50-4.95 (m, 2H, NC*H*₂C(O)); 6.90 (m, br, 1H, C(O)N*H*CH₂); 7.04 (d, 2H, *Ph*); 7.30 (d, 1H, CC*H*=CHN); 8.00 (d, 1H, CCH=C*H*N); 8.03 (d, 2H, *Ph).*
**T^{R}:**
   **¹H-NMR**(DMSO-d₆): 1.22 (t, 6H, P(O)(OCH₂C*H*₃)₂); 1.39 (s, 9H, C(C*H*₃)₃); 1.65-2.20 (m, 4H, CHC*H*₂C*H*₂P); 1.75 (s, 3H, C=CC*H*₃); 2.90-3.50 (m, 4H, NC*H*₂C*H*₂N); 3.90-4.10 (m, 4H, P(O)(OC*H*₂CH₃)₂); 4.18 (m, 1H, NC*H*C(O)); 4.45-4.65 (m, 2H, NC*H*₂C(O)); 6.86 (m, br, 1H, C(O)N*H*CH₂); 7.37 (s, 1H, NC*H*=C).
**P^{R}:**
   **¹H-NMR**(DMSO-d₆): 1.20 (t, 6H, P(O)(OCH₂C*H*₃)₂); 1. 38 (s, 9H, C(C*H*₃)₃); 1.46-2.30 (m, 4H, CHC*H*₂C*H*₂P); 3.00-3.45 (m, 4H, NC*H*₂C*H*₂N); 3.50-3.75 (m, 2H, CC*H*₂C(O)); 3.80-4.00 (m, 4H, P(O)(OC*H*₂CH₃)₂); 4.22 (m, 1H, NC*H*C(O)); 7.10-7.30 (m, 5H, *Ph*)*.*

### Beispiel 10: Herstellung von weiteren Verbindungen der allgemeinen Formel II mit S-Konfiguration am asymmetrischen Zentrum:

Das Herstellungsverfahren für die Verbindungen der allgemeinen Formel **II** mit der R-Konfiguration wird analog für die Herstellung der entsprechenden Verbindungen der allgemeinen Formel **II** mit S-Konfiguration angewendet. Hierbei wird bei der in Beispiel 1 beschriebenen Synthese das (R)-2,5-Dihydro-3,6-dimethoxy-2-isopropylpyrazin als Edukt eingesetzt und die folgenden Synthesen analog wie beschrieben durchgeführt.

Beispielsweise wurde hergestellt:
**J^{S}(Z)** :
   **¹H-NMR**(DMSO-d₆): 1.32 (t, 6H, P(O)(OCH₂C*H*₃)₂), 1.42 (s, 9H, C(C*H*₃)₃); 1.60-2.50 (m, 4H, CHC*H*₂C*H*₂P,), 3.10-3.55 (m, 4H, NC*H*₂C*H*₂N); 3.65-3.90 (m, 2H, NC*H*₂C(O)); 4.00-4.15 (m, 4H, P(O)(OC*H*₂CH₃)₂); 4.20 (m, 1H, NC*H*C(O)); 5. 24 (s, 2H, OC*H*₂Ph); 6.80 (m, br, 1H, C(O)N*H*CH₂); 7.27 (d, 1H, C=CHN); 7.30-7.50 (m, 5H, *Ph*)*.*

### Beispiel 11: Allgemeine Synthesevorschrift für erfindungsgemäße Verbindungen mit MNPA-Substituent am Rest K:

Durch sequenzielle Verknüpfung von entsprechenden Verbindungen der allgemeinen Formel **II** mit asymmetrischem Zentrum und/oder entsprechender Verbindungen der allgemeinen Formel II ohne asymmetrisches Zentrum und/oder Aminosäuren und/oder Aminosäurederivaten und/oder Fluoreszenzmarkern mittels Festphasenpeptidsynthese werden die erfindungsgemäßen Verbindungen hergestellt.

Dabei wird folgendes Syntheseprotokoll verwendet:
Schritt 1: 3 h Vorquellen von 10 mg Harz (Boc-Gly-PAM-MBHA, 0,54 mmol/g) in Dichlormethan.
Schritt 2: Beginn des Synthesezyklus: 4x Waschen mit Dichlormethan.
Schritt 3: Boc-Abspaltung durch Reaktion mit TFA/m-Kresol (95:5). Reaktionsdauer: 2x je 3 min
Schritt 4: 5x Waschen mit Dichlormethan.
Schritt 5: 5x Waschen mit NMP.
Schritt 6: 1 min Voraktivierung von 4 Äquivalenten der entsprechenden geschützten Verbindung der allgemeinen Formel **II** bzw. einer entsprechend geschützten Aminosäure mit 3,8 Äquivalenten HATU und 9 Äquivalenten NMM in NMP/Pyridin (2:1). Schritt 7: Reaktion der aktivierten geschützten Verbindung der allgemeinen Formel **II** bzw. einer entsprechend geschützten Aminosäure mit der festen Phase (1. Kupplung; Dauer: 30 min). Schritt 8: 4x Waschen mit NMP.
Schritt 9: 1x Waschen mit Dichlormethan.
Schritt 10: Wiederholung der Schritte 6 bis 8 (2.Kupplung). Schritt 11: Überprüfung der Kupplungseffizienz mit Ninhydrin (Kaiser-Test; Wenn der Kaiser-Test positiv ist, müssen die Schritte 6 bis 8 mit der entsprechenden geschützten Verbindung der allgemeinen Formel II wiederholt werden).
Schritt 12: Nach negativem Kaiser-Test, 2x Capping mit einer Lösung aus Ac₂O/NMP/Pyridin (1:25:25) für je 4 min.
Schritt 13: 5x Waschen mit NMP.
Schritt 14: Wiederholen des Synthesezyklus (Schritte 2 bis 13) bis zur Kupplung mit der letzten entsprechenden geschützten Verbindung der allgemeinen Formel **II.** Anschließend gegebenenfalls Wiederholen des Synthesezyklus (Schritte 2 bis 13) bis zur Kupplung mit der letzten entsprechend geschützten Aminosäure. Schritt 15: 4x Waschen mit Dichlormethan.
Schritt 16: Boc-Abspaltung durch Reaktion mit TFA/m-Kresol (95:5). Reaktionsdauer: 2x je 3 min
Schritt 17: 5x Waschen mit Dichlormethan.
Schritt 18: 5x Waschen mit NMP.
Schritt 19: 1 min Voraktivierung von 6 Äquivalenten MNPA-OH mit 5,7 Äquivalenten HATU und 13 Äquivalenten NMM in NMP/Pyridin (2:1).
Schritt 20: Reaktion von aktivierten MNPA-OH mit der festen Phase (Dauer: 30 min).
Schritt 21: 4x Waschen mit NMP.
Schritt 22: Wiederholung der Schritte 19 bis 21 (2.Kupplung).
Schritt 23: 5x Waschen mit Dichlormethan.
Schritt 24: zur Trocknung: 5x Waschen mit Diethylether.

Man erhält eine Verbindung der allgemeinen Formel I, die am C-terminalen Ende an das Harz gebunden ist.

Abspaltung der erfindungsgemäßen Verbindung vom Harz:
Das Harz mit der erfindungsgemäßen Verbindung wird in wässriger Ammoniaklösung (28-30 Gewichtsprozente NH₃ in H₂O) bei 60 °C 20 h gerührt. Das abgespaltene Harz wird anschließend abfiltriert und
das Filtrat im Vakuum eingeengt und getrocknet. Das Rohprodukt wird durch präparative HPLC über eine RP-C18-Säule mit Methanol / Wasser gereinigt. Man erhält die erfindungsgemäße Verbindung als farblosen Feststoff in ca. 50%-iger Ausbeute. Die Masse der erfindungsgemäßen Verbindung wird mit MALDI-TOF charakterisiert.

### Beispiel 12: Allgemeine Synthesevorschrift für erfindungsgemäße Verbindungen mit DNPA-Substituent am Rest K:

Durch sequenzielle Verknüpfung von entsprechenden Verbindungen der allgemeinen Formel **II** mit asymmetrischem Zentrum und/oder entsprechender Verbindungen der allgemeinen Formel **II** ohne asymmetrisches Zentrum und/oder Aminosäuren und/oder Aminosäurederivaten und/oder Fluoreszenzmarkern mittels Festphasenpeptidsynthese werden die erfindungsgemäßen Verbindungen hergestellt.

Dabei wird folgendes Syntheseprotokoll verwendet:
Schritt 1: 3 h Vorquellen von 10 mg Harz (Boc-Gly-PAM-MBHA, 0,54 mmol/g) in Dichlormethan.
Schritt 2: Beginn des Synthesezyklus: 4x Waschen mit Dichlormethan.
Schritt 3: Boc-Abspaltung durch Reaktion mit TFA/m-Kresol (95:5). Reaktionsdauer: 2x je 3 min
Schritt 4: 5x Waschen mit Dichlormethan.
Schritt 5: 5x Waschen mit NMP.
Schritt 6: 1 min Voraktivierung von 4 Äquivalenten der entsprechenden geschützten Verbindung der allgemeinen Formel **II** bzw. einer entsprechend geschützten Aminosäure mit 3,8 Äquivalenten HATU und 9 Äquivalenten NMM in NMP/Pyridin (2:1).
Schritt 7: Reaktion der aktivierten geschützten Verbindung der allgemeinen Formel **II** bzw. einer entsprechend geschützten Aminosäure mit der festen Phase (1. Kupplung; Dauer: 30 min).
Schritt 8: 4x Waschen mit NMP.
Schritt 9: 1x Waschen mit Dichlormethan.
Schritt 10: Wiederholung der Schritte 6 bis 8 (2.Kupplung).
Schritt 11: Überprüfung der Kupplungseffizienz mit Ninhydrin (Kaiser-Test; Wenn der Kaiser-Test positiv ist, müssen die Schritte 6 bis 8 mit der entsprechenden geschützten Verbindung der allgemeinen Formel **II** wiederholt werden).
Schritt 12: Nach negativem Kaiser-Test, 2x Capping mit einer Lösung aus Ac₂O/NMP/Pyridin (1:25:25) für je 4 min.
Schritt 13: 5x Waschen mit NMP.
Schritt 14: Wiederholen des Synthesezyklus (Schritte 2 bis 13) bis zur Kupplung mit der letzten entsprechenden geschützten Verbindung der allgemeinen Formel **II.** Anschließend gegebenenfalls Wiederholen des Synthesezyklus (Schritte 2 bis 13) bis zur Kupplung mit der letzten entsprechend geschützten Aminosäure.
Schritt 15: 4x Waschen mit Dichlormethan.
Schritt 16: Boc-Abspaltung durch Reaktion mit TFA/m-Kresol (95:5). Reaktionsdauer: 2x je 3 min
Schritt 17: 5x Waschen mit Dichlormethan.
Schritt 18: 5x Waschen mit NMP.
Schritt 19: 1 min Voraktivierung von 6 Äquivalenten DNPA-OH mit 5,7 Äquivalenten HATU und 13 Äquivalenten NMM in NMP/Pyridin (2:1).
Schritt 20: Reaktion von aktivierten DNPA-OH mit der festen Phase (Dauer: 30 min).
Schritt 21: 4x Waschen mit NMP.
Schritt 22: Wiederholung der Schritte 19 bis 21 (2.Kupplung). Schritt 23: 5x Waschen mit Dichlormethan.
Schritt 24: zur Trocknung: 5x Waschen mit Diethylether.

Man erhält eine Verbindung der allgemeinen Formel I, die am C-terminalen Ende an das Harz gebunden ist.

Abspaltung der erfindungsgemäßen Verbindung vom Harz:
Das Harz mit der erfindungsgemäßen Verbindung wird in wässriger Ammoniaklösung (28-30 Gewichtsprozente NH₃ in H₂O) bei 60 °C 20 h gerührt. Das abgespaltene Harz wird anschließend abfiltriert und
das Filtrat im Vakuum eingeengt und getrocknet. Das Rohprodukt wird durch präparative HPLC über eine RP-C18-Säule mit Methanol / Wasser gereinigt. Man erhält die erfindungsgemäße Verbindung als farblosen Feststoff in ca. 50%-iger Ausbeute. Die Masse der erfindungsgemäßen Verbindung wird mit MALDI-TOF charakterisiert.

### Beispiel 13: Allgemeine Synthesevorschrift für die erfindungsgemäBen Verbindungen mit Linker und NNPA-Substituent bzw. DNPA-Substituent am Rest K:

Durch sequenzielle Verknüpfung von entsprechenden Verbindungen der allgemeinen Formel **II** mit asymmetrischem Zentrum und/oder entsprechender Verbindungen der allgemeinen Formel **II** ohne asymmetrisches Zentrum und/oder Aminosäuren und/oder Aminosäurederivaten und/oder Fluoreszenzmarkern sowie geeigneten Linker-Monomeren mittels Festphasenpeptidsynthese werden die erfindungsgemäßen Verbindungen hergestellt.

Dabei wird folgendes Syntheseprotokoll verwendet:

### Syntheseprotokoll:

Schritt 1: 3 h Vorquellen von 10 mg Harz (Boc-Gly-PAM-MBHA, 0,54 mmol/g) in Dichlormethan.
Schritt 2: Beginn des Synthesezyklus: 4x Waschen mit Dichlormethan.
Schritt 3: Boc-Abspaltung durch Reaktion mit TFA/m-Kresol (95:5). Reaktionsdauer: 2x je 3 min
Schritt 4: 5x Waschen mit Dichlormethan.
Schritt 5: 5x Waschen mit NMP.
Schritt 6: 1 min Voraktivierung von 4 Äquivalenten der entsprechenden geschützten Verbindung der allgemeinen Formel **II** bzw. einer entsprechend geschützten Aminosäure mit 3,8 Äquivalenten HATU und 9 Äquivalenten NMM in NMP/Pyridin (2:1).
Schritt 7: Reaktion der entsprechenden geschützten Verbindung der allgemeinen Formel **II** bzw. einer entsprechend geschützten Aminosäure mit der festen Phase (1. Kupplung; Dauer: 30 min).
Schritt 8: 4x Waschen mit NMP.
Schritt 9: 1x Waschen mit Dichlormethan.
Schritt 10: Wiederholung der Schritte 6 bis 8 (2.Kupplung).
Schritt 11: Überprüfung der Kupplungseffizienz mit Ninhydrin (Kaiser-Test; Wenn der Kaiser-Test positiv ist, müssen die Schritte 6 bis 8 mit der entsprechenden geschützten Verbindung der allgemeinen Formel **II** wiederholt werden).
Schritt 12: Nach negativem Kaiser-Test, 2x Capping mit einer Lösung aus Ac₂O/NMP/Pyridin (1:25:25) für je 4 min.
Schritt 13: 5x Waschen mit NMP.
Schritt 14: Wiederholen des Synthesezyklus (Schritte 2 bis 13) bis zur Kupplung der Linker eg1 (8-Amino-2,6-dioxaoktansäure).
Schritt 15: Kupplung der Linker: 4x Waschen mit Dichlormethan.
Schritt 16: Boc-Abspaltung durch Reaktion mit TFA/m-Kresol (95:5). Reaktionsdauer: 2x je 3 min
Schritt 17: 5x Waschen mit Dichlormethan.
Schritt 18: 5x Waschen mit NMP.
Schritt 19: 1 min Voraktivierung von 4 Äquivalenten eg1 mit 3,8 Äquivalenten HATU und 9 Äquivalenten NMM in NMP/Pyridin (2:1). Schritt 20: Reaktion des aktivierten Linkers mit der festen Phase (1. Kupplung; Dauer: 30 min).
Schritt 21: 4x Waschen mit NMP.
Schritt 22: 1x Waschen mit Dichlormethan.
Schritt 23: Wiederholung der Schritte 19 bis 21 (2.Kupplung).
Schritt 24: Überprüfung der Kupplungseffizienz mit Ninhydrin (Kaiser-Test; Wenn der Kaiser-Test positiv ist, müssen die Schritte 19 bis 21 wiederholt werden).
Schritt 25: 2x Capping mit einer Lösung aus Ac₂O/NMP/Pyridin (1:25:25) für je 4 min nach negativem Kaiser-Test.
Schritt 26: 5x Waschen mit NMP.
Schritt 27: 2x Wiederholen des Syntheseabschnitts (Schritte 15 bis 26) für (eg1)₃.
Schritt 28: Wiederholen des Synthesezyklus (Schritte 2 bis 13) bis zur Kupplung mit der letzten entsprechenden geschützten Verbindung der allgemeinen Formel **II.** Anschließend gegebenenfalls Wiederholen des Synthesezyklus (Schritte 2 bis 13) bis zur Kupplung mit der letzten entsprechend geschützten Aminosäure. Anschließend die Durchführung der Schritte 15-24 aus Beispiel 11 im Falle der Kupplung MNPA-OH bzw. die Durchführung der Schritte 15-24 aus Beispiel 12 im Falle der Kupplung DNPA-OH.

Man erhält eine erfindungsgemäße Verbindung mit Linker, die am C-terminalen Ende an das Harz gebunden ist.

Abspaltung der erfindungsgemäßen Verbindung mit Linker vom Harz: Das Harz mit der erfindungsgemäßen Verbindung mit Linker wird in wässriger Ammoniaklösung (28-30 Gewichtsprozente NH₃ in H₂O) bei 60 °C 20 h gerührt. Das abgespaltene Harz wird anschließend abfiltriert und das Filtrat im Vakuum eingeengt und getrocknet. Das Rohprodukt wird durch präparative HPLC über eine RP-C18-Säule mit Methanol / Wasser gereinigt. Man erhält die erfindungsgemäße Verbindung mit Linker als farblosen Feststoff in 50%-iger Ausbeute. Die Masse der erfindungsgemäßen Verbindung mit Linker wird mit MALDI-TOF charakterisiert.

### Beispiel 14: Weitere Beispiele von Sequenzen

Durch Durchführung der allgemeinen Synthesevorschriften aus den Beispielen 11 oder 12 wurden weitere erfindungsgemäße Verbindungen hergestellt:
MNPA-A^{R}cG^{R}gT^{R}cG^{R}gC^{R}gA^{R}aC^{R}aT^{R}-Gly-NH₂
MNPA-Bio-A^{R}cG^{R}gT^{R}cG^{R}gC^{R}gA^{R}aC^{R}aT^{R}-Gly-NH₂ (Bio = Lysin,
funktionalisiert mit Biotin über die Aminfunktion der Lysin-Seitenkette)
DNPA-A^{R}cG^{R}gT^{R}cG^{R}gC^{R}gA^{R}aC^{R}aT^{R}-Gly-NH₂
DNPA-Bio-A^{R}cG^{R}gT^{R}cG^{R}gC^{R}gA^{R}aC^{R}aT^{R}-Gly-NH₂
DNPA-tG^{R}cC^{R}tA^{R}gG^{R}aC^{R}tC^{R}cA^{R}gC^{R}-Gly-NH₂
DNPA-Bio-tG^{R}CC^{R}tA^{R}gG^{R}aC^{R}tC^{R}CA^{R}gC^{A}-Gly-NH₂
DNPA-tG^{R}cC^{R}tA^{R}ggactC^{R}cA^{R}gC^{R}-Gly-NH₂
DNPA-Bio-tG^{R}cC^{R}tA^{R}ggactC^{R}cA^{R}gC^{R}-Gly-NH₂
DNPA-cG^{R}aA^{R}tA^{R}aG^{R}gA^{R}gG^{R}cT^{R}tA^{R}-Gly-NH₂
DNPA-Bio-cG^{R}aA^{R}tA^{R}aG^{R}gA^{R}gG^{R}CT^{R}tA^{R}-Gly-NH₂
DNPA-cG^{R}aA^{R}tA^{R}aggagG^{R}cT^{R}tA^{R}-Gly-NH₂
DNPA-Bio-cG^{R}aA^{R}tA^{R}aggagG^{R}CT^{R}tA^{R}-Gly-NH₂
DNPA-gG^{R}cT^{R}cG^{R}aA^{R}tA^{R}aG^{R}gA^{R}gG^{R}-Gly-NH₂
DNPA-Bio-gG^{R}cT^{R}cG^{R}aA^{R}tA^{R}aG^{R}gA^{R}gG^{R}-Gly-NH₂
DNPA-gG^{R}cT^{R}cG^{R}aataaG^{R}gA^{R}gG^{R}-Gly-NH₂
DNPA-Bio-gG^{R}cT^{R}cG^{R}aataaG^{R}gA^{R}gG^{R}-Gly-NH₂
DNPA-aC^{R}aA^{R}aT^{R}gC^{R}aT^{R}gG^{R}gC^{R}tG^{R}-Gly-NH₂
DNPA-Bio-aC^{R}aA^{R}aT^{R}gC^{R}aT^{R}gG^{R}gC^{R}tG^{R}-Gly-NH₂
DNPA-aC^{R}aA^{R}aT^{R}gcatgG^{R}gC^{R}tG^{R}-Gly-NH₂
DNPA-Bio-aC^{R}aA^{R}aT^{R}gcatgG^{R}gC^{R}tG^{R}-Gly-NH₂
DNPA-cG^{R}cC^{R}tT^{R}aT^{R}cC^{R}gT^{R}aG^{R}cC^{R}-Gly-NH₂
DNPA-Bio-cG^{R}cC^{R}tT^{R}aT^{R}cC^{R}gT^{R}aG^{R}cC^{R}-Gly-NH₂
DN PA-cG^{R}cC^{R}tT^{R}atccgT^{R}aG^{R}cC^{R}-Gly-NH₂
DNPA-Bio-cG^{R}cC^{R}tT^{R}atccgT^{R}aG^{R}cC^{R}-Gly-NH₂
DNPA-tgccT^{R}aG^{R}gactcC^{R}aG^{R}c-Gly-NH₂

DNPA-Dota-gG^{R}cT^{R}cG^{R}aA^{R}tA^{R}aG^{R}gA^{R}gG^{R}-Gly-NH₂ (DOTA= Lysin, funktionalisiert mit DOTA über die Aminfunktion der Lysin-Seitenkette)

### Beispiel 15: Synthesevorschrift für eine erfindungsgemäße Verbindungen der allgemeinen Formel V:

In einem Schraubdeckelglas werden 2ml DMF und 2 ml Pyridin vorgelegt. Unter Rühren werden 382 mg (2.95 mmol) DIPEA und anschließend 291 mg (1.48 mmol) 4-Hydroxy-3-nitro-phenylessigsäure zugefügt. Anschließend werden 562 mg (1.48 mmol) HATU, gelöst in 2 ml DMF, zugefügt. Man lässt 5 min voraktivieren. Die voraktivierte Lösung wird zu einer Lösung aus 500 mg (1.48 mmol) 2-(10-Hydroxydecyl)-5,6-dimethoxy-3-methylcyclohexa-2,5-diene-1,4-dione (Idebenone), gelöst in 10 ml DMF, zugetropft und anschließend auf 40 °C erhitzt. Nach 24 Stunden Reaktionszeit wird das Lösungsmittel entfernt und der Rückstand in Essigester aufgenommen. Die organische Phase wird zweimal mit 2 N Kaliumhydrogensulfatlösung und einmal mit gesättigter Kochsalzlösung gewaschen und anschließend über Magnesiumsulfat getrocknet. Das Rohprodukt wird durch Chromatographie (Kieselgel, Hexan/Essigester, 1/1, v/v) gereinigt.

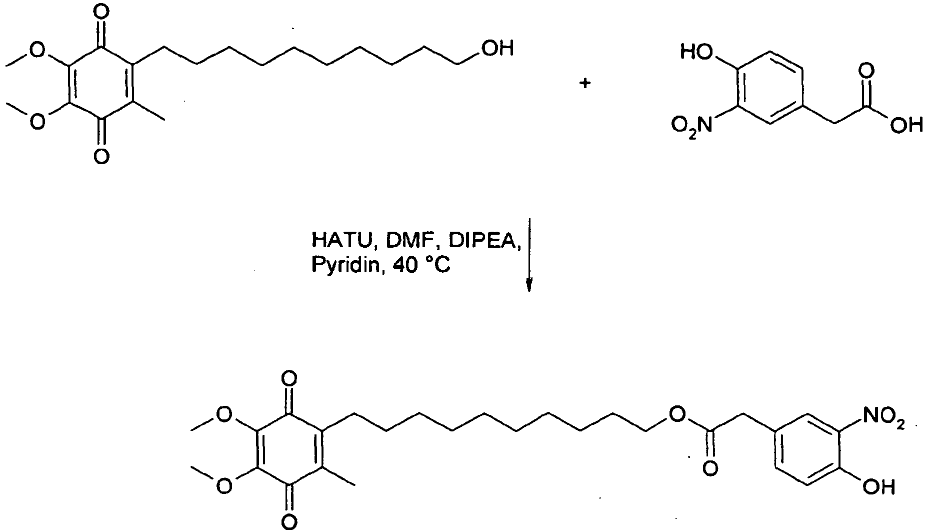

### Beispiel 16: Selektive Lokalisierung von Verbindungen der allgemeinen Formel I, die einen Monohydroxy-mononitro-phenyl-Rest bzw. Monohydroxy-dinitro-phenyl-Rest tragen.

Hela-Zellen bzw. 143B parentale Zellen werden mit einer 10µM Lösung von Biotin-gelabelten Verbindungen der allgemeinen Formel I inkubiert. Nach 24 Stunden wird eine 2µM MitoTracker-Lösung dazugegeben, und nach weiteren 45 Minuten werden die Zellen mit Ethanol fixiert. Anschließend lässt man auf die Zellen 30 Minuten lang eine Fluorescein-Avidin-Lösung (5µg/ml) bei Raumtemperatur einwirken. Nach dem Waschen der Zellen lässt man 30 Minuten lang eine biotinylierte Anti-Avidin Lösung (5µg/ml) auf die Zellen bei Raumtemperatur einwirken. Nach erneutem Waschen der Zellen lässt man auf die Zellen wiederum 30 Minuten lang eine Fluorescein-Avidin-Lösung (5µg/ml) bei Raumtemperatur einwirken. Nach weiteren Waschschritten wird der Zellkern durch DAPI-Gegenfärbung markiert. Anschließend wird die räumliche Verteilung bzw. Ausbreitung der Verbindungen der allgemeinen Formel I, der Mitochondrien sowie des Zellkerns innerhalb der Zellen mit einem konfokalen Mikroskop untersucht.

### Beispiel 17: Reduktion von COX1 und Menge an mtDNA in HeLa-Zellen durch Behandlung mit der erfindungsgemäßen Verbindung DNPA-tG^{R}cC^{R}tA^{R}ggactC^{R}cA^{R}gC^{R}-Gly-NH₂.

HeLa-Zellen werden mit einer Lösung von DNPA-tG^{R}cC^{R}tA^{R}ggactC^{R}cA^{R}gC^{R} -Gly-NH₂ ("Effektiv"), das gegen eine Position auf der mtDNA /mtRNA gerichtet ist, welche für das mitochondriale Protein COX1 codiert, in verschiedenen Konzentrationen (100nM, 250nM, 500nM, 1µM, 2,5µM, 5µM and 10µM) und für unterschiedliche Zeiträume inkubiert (3, 6, 9, 11 und 17 Tagen). Bei Experimenten, die länger als 3 Tage dauern, wird der Überstand alle drei Tage durch frisches Medium mit jeweils gleicher Konzentration an der erfindungsgemäßen Verbindung DNPA-tG^{R}cC^{R}tA^{R}ggactC^{R}cA^{R}gC^{R}-Gly-NH₂ ersetzt.

Die Bestimmung der COX1-Level erfolgt durch Western-Blotting gegen Porin, ein mitochondriales Transmembran-Protein, dessen Gehalt nicht durch die Behandlung mit der erfindungsgemäßen Verbindung beeinflusst wird, als internem Standard. Die Reduktion von COX1 wird im Vergleich zu den unbehandelten HeLa-Zellen dargestellt. Dabei wird das COX1-Level der unbehandelten HeLa-Zellen als 100% definiert.

Bei einer Konzentration von 10µM an erfindungsgemäßer Verbindung ist das COX1-Level in den HeLa-Zellen nach 3 Tagen auf 67% und nach 9 Tagen auf 20% reduziert.

Die folgende Tabelle zeigt die Konzentrationsabhängigkeit der COX1-Reduktion nach einer Behandlungsdauer von 9 Tagen:

| Konzentration [µM] | 10 | 5 | 2,5 | 1 | 0,5 | 0,25 | 0,1 |
|---|---|---|---|---|---|---|---|
| COX1 [%] | 20 | 48 | 55 | 75 | 80 | 88 | 100 |

Die Bestimmung der mtDNA-Menge erfolgt durch Real-Time-PCR gegen Zellkern-DNA, die nicht durch die Behandlung mit der erfindungsgemäßen Verbindung DNPA-tG^{R}cC^{R}tA^{R}ggactC^{R}cA^{R}gC^{R}-Gly-NH₂ beeinflusst wird, als internem Standard. Die Reduktion von mtDNA wird im Vergleich zu den unbehandelten HeLa-Zellen dargestellt. Dabei wird die Menge an mtDNA der unbehandelten HeLa-Zellen als 100% definiert.

Als weiterer Vergleich wurde eine erfindungsgemäße Verbindung ohne komplementäre Sequenz zu mtDNA / mtRNA verwendet ("negative Kontrolle")

Bei einer Konzentration an erfindungsgemäßer Verbindung DNPA-tG^{R}cC^{R}tA^{R}ggactC^{R}cA^{R}gC^{R}-Gly-NH₂ von 10µM kann nach 3 Tagen noch kein Effekt festgestellt werden. Nach 6 Tagen ist, im Vergleich zu unbehandelten Hela-Zellen bzw. im Vergleich zu HeLa-Zellen, die mit der negativen Kontrolle behandelt wurden, eine Reduktion der mtDNA auf 81% und nach 9 Tagen auf 62% beobachtbar. Danach bleibt der Wert der reduzierten mtDNA-Menge auch nach 11 Tagen (64%) und 17 Tagen (61%) konstant.

Die folgende Tabelle zeigt die Konzentrationsabhängigkeit der mtDNA-Reduktion nach einer Behandlung mit der effektiven erfindungsgemäßen Verbindung von 9 Tagen im Vergleich zu der negativen Kontrolle:

| | | | | | | | |
|---|---|---|---|---|---|---|---|
| Konzentration [µM] | 10 | 5 | 2,5 | 1 | 0,5 | 0,25 | 0,1 |
| mtDNA [%] "Effektiv" | 62 | 60 | 82 | 81 | 84 | 101 | 97 |
| mtDNA [%] "negative Kontrolle" | 93 | 101 | 99 | 97 | 110 | 94 | 94 |

## Patentansprüche

1. Verbindung der Formel I, 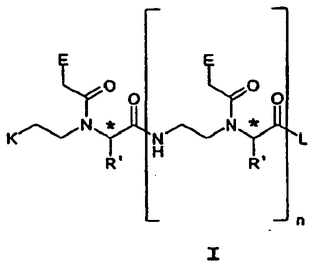 wobei
n eine ganze Zahl von 0 bis 35 ist,
jedes E unabhängig voneinander ein H-Atom, ein substituierter oder unsubstituierter Phenylrest, ein substituierter oder unsubstituierter Heterocyclus, eine gegebenenfalls mit Schutzgruppen substituierte Nukleobase, oder ein DNA-Intercalator ist,
jedes R¹ unabhängig voneinander ein H-Atom oder eine Seitenkette einer natürlichen oder nichtnatürlichen Aminosäure, oder ein gegebenenfalls substituierter Alkyl-, Alkenyl-, Alkylaryl-, Aryl-, heterocyclischer oder alicyclischer Rest mit bis zu 20 C-Atomen ist, wobei mindestens einer der gegebenenfalls substituierten Alkyl-, Alkenyl-, Alkylaryl-, Aryl-, heterocyclischen oder alicyclischen Reste mit bis zu 20 C-Atomen mit einer oder mehreren Phosphonsäureester- oder Phosphonsäure-Funktionen substituiert ist,
K eine Gruppe der Formel -NR²R³, -N^{⊕}R²R³R⁴, -NR²(CO)R³ oder-NR²(CS)R³ ist, wobei R², R³ und R⁴ unabhängig voneinander ein H-Atom, ein Alkyl-, Alkaryl-, Alkenyl, oder Alkinyl- Rest, Amino-Schutzgruppe, Reporterligand, Fluoreszenz-Marker, Intercalator, Chelator, Aminosäure, Peptid, Protein, Kohlenhydrat, Lipid, Steroid, Fettsäure, Oligonukleotid, Quantum-Dot, FRET-Quencher (Fluoreszenz-Resonanz-Energie-Transfer-Quencher) oder ein in Wasser lösliches oder unlösliches Polymer sind, wobei jeder der vorstehenden Reste gegebenenfalls substituiert sein kann,
L eine Gruppe der Formel -NR⁵R⁶, -NR⁵(CO)R⁶, -NR⁵(CS)R⁶, -OR⁷ oder -SR⁷ ist, wobei R⁵ und R⁶ unabhängig voneinander ein H-Atom, ein Alkyl-, Alkaryl-, Alkenyl, oder Alkinyl- Rest, Reporterligand, Fluoreszenz-Marker, Intercalator, Chelator, Aminosäure, Aminosäureamid, Peptid, Peptidamid, Protein, Kohlenhydrat, Lipid, Steroid, Fettsäure, Oligonukleotid , Quantum-Dot, FRET-Quencher (Fluoreszenz-Resonanz-Energie-Transfer-Quencher) oder ein in Wasser lösliches oder ein unlösliches Polymer und R⁷ ein H-Atom, ein Alkylrest, Reporterligand, Fluoreszenz-Marker, Intercalator, Chelator, Aminosäure, Aminosäureamid, Peptid, Peptidamid, Protein, Kohlenhydrat, Lipid, Steroid, Fettsäure, Oligonukleotid, Quantum-Dot, FRET-Quencher oder ein in Wasser lösliches oder ein unlösliches Polymer ist, wobei jeder der vorstehenden Reste gegebenenfalls substituiert sein kann,
wobei die Reste K, L oder R¹ unabhängig voneinander mit mindestens einem Monohydroxy-mononitro-phenyl-Rest oder Monohydroxy-dinitro-phenyl-Rest substituiert sind.

2. Verbindung nach Anspruch 1, wobei die Verbindung nach Formel I mindestens 1 asymmetrisches Zentrum aufweist, und vorzugsweise

3. Verbindung nach einem der vorhergehenden Ansprüche, wobei jeder zweite oder jeder dritte Rest R¹ unabhängig voneinander ein gegebenenfalls substituierter Alkyl-, Alkenyl-, Alkylaryl-, Aryl-, heterocyclischer oder alicyclischer Rest mit bis zu 20 C-Atomen ist und die übrigen Reste R¹ H-Atome sind.

4. Verbindung nach einem der vorstehenden Ansprüche, wobei zwei, drei oder mehr benachbarte Reste R¹ unabhängig voneinander gegebenenfalls substituierte Alkyl-, Alkenyl-, Alkylaryl-, Aryl-, heterocyclischer oder alicyclischer Reste mit bis zu 20 C-Atomen sind und die übrigen Reste R¹ H-Atome sind.

5. Verbindung nach einem der Ansprüche 1 bis 2, wobei jedes R¹ unabhängig voneinander ein gegebenenfalls substituierter Alkyl-, Alkenyl-, Alkylaryl-, Aryl-, heterocyclischer oder alicyclischer Rest mit bis zu 20 C-Atomen ist.

6. Verbindung nach einem der vorhergehenden Ansprüche, wobei einer oder mehrere der Reste R¹ unabhängig voneinander Phosphonsäureester- oder Phosphonsäure-Funktionen aufweist.

7. Verbindung nach einem der vorhergehenden Ansprüche, wobei alle asymmetrischen Zentren die gleiche Konfiguration aufweisen, insbesondere wobei alle asymmetrischen Zentren (S)-Konfiguration aufweisen, oder wobei alle asymmetrischen Zentren (R)-Konfiguration aufweisen.

8. Verbindung nach einem der vorhergehenden Ansprüche, wobei jedes R¹ unabhängig voneinander eine oder mehrere Phosphonsäureester- oder Phosphonsäure-Funktionen aufweist, wobei die Phosphonsäureester-Funktionen die Formel -P(=O)(OV)₂ oder -P(=O)(OV)(OH) besitzen, wobei jedes V unabhängig voneinander ein unsubstituierter Alkyl-, Alkenyl-, Alkylaryl-, Aryl- oder alicyclischer Rest mit bis zu 20 C-Atomen ist, insbesondere wobei jedes V unabhängig voneinander ein Methyl-, Ethyl-, Cyclohexyl- oder Benzyl-Rest ist.

9. Verbindung, die mindestens zwei Verbindungen nach einem der Ansprüche 1 bis 8 enthält, welche über einen Linker miteinander verbunden sind, wobei der Linker vorzugsweise eine Alkylkette, ein Peptid, ein Oligonukleotid oder ein Oligomer ist, das aus mindestens drei 8-Amino-3,6-dioxaoctansäure-Einheiten aufgebaut ist.

10. Zusammensetzung, die mindestens eine Verbindung nach einem der vorstehenden Ansprüche enthält, insbesondere Pharmazeutische Zusammensetzung, die mindestens eine Verbindung nach einem der Ansprüche 1 bis 9, gegebenenfalls in Kombination mit mindestens einem Träger, Lösungsmittel oder sonstigen pharmazeutischen Hilfsstoff, enthält.

11. Verwendung einer Verbindung oder Zusammensetzung nach einem der Ansprüche 1 bis 10 für die Herstellung eines Arzneimittels zur Behandlung oder Prophylaxe von mitochondrialen Erkrankungen.

12. Verwendung einer Verbindung oder Zusammensetzung nach einem der Ansprüche 1 bis 10 für die Herstellung eines Mittels zur Diagnose von mitochondrialen Eigenschaften oder mitochondrialen Erkrankungen.

13. Verwendung nach einem der Ansprüche 11 bis 12, wobei die mitochondriale Erkrankung eine Erbkrankheit ist.

14. Verwendung nach einem der Ansprüche 11 bis 13 zur Behandlung oder Prophylaxe von Krebs, der Parkinsonschen Krankheit, von Phänomenen des Alterns, oder von Arteriosklerose.

15. Verwendung einer Verbindung oder Zusammensetzung nach einem der Ansprüche 1 bis 10 für die Herstellung eines Arzneimittels zur Verhinderung der mtDNA-Transkription in Zellen oder in lebenden Organismen, oder für die Herstellung eines Arzneimittels zur Verhinderung der Translation von mtRNA in Zellen oder in lebenden Organismen.

## Claims

1. A compound of the formula I, 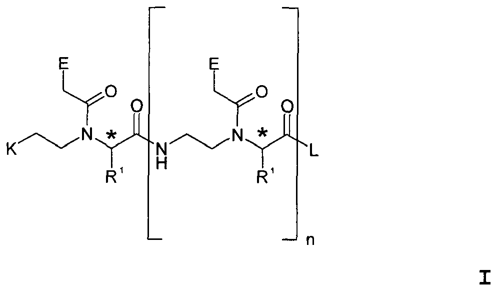 wherein
n represents an integer from 0 to 35,
each E independently of each other represents a hydrogen atom, a substituted or unsubstituted phenyl group, a substituted or unsubstituted heterocycle, a nucleobase, optionally substituted with protecting groups, or a DNA intercalator,
each R¹ independently of each other represents a hydrogen atom or a side chain of a naturally occurring or non-naturally occurring amino acid, or an optionally substituted alkyl, alkenyl, alkylaryl, aryl, heterocyclic or alicyclic group having up to 20 carbon atoms, wherein at least one of the optionally substituted alkyl, alkenyl, alkylaryl, aryl, heterocyclic or alicyclic groups having up to 20 carbon atoms is substituted with one or more phosphonic acid ester functions or phosphononic acid functions,
K represents a group of the formula -NR²R³, -N^{⊕}R²R³R⁴, -NR²(CO)R³ or -NR²(CS)R³, wherein R², R³ and R⁴ independently of each other represent a hydrogen atom, an alkyl, alkaryl, alkenyl, or alkinyl rest, amino protecting group, reporter ligand, fluorescence marker, intercalator, chelator, amino acid, peptide, protein, carbohydrate, lipid, steroid, fatty acid, oligonucleotide, quantum dot, FRET quencher (fluorescence resonance energy transfer quencher) or a polymer soluble or insoluble in water, wherein each of the above mentioned groups optionally may be substituted,
L represents a group of the formula -NR⁵R⁶, -NR⁵(CO)R⁶,-NR⁵(CS)R⁶, -OR⁷ or -SR⁷, wherein R⁵ and R⁶ independently of each other represent a hydrogen atom, an alkyl, alkaryl, alkenyl, or alkinyl group, reporter ligand, fluorescence marker, intercalator, chelator, amino acid, amino acid amide, peptide, peptide amide, protein, carbohydrate, lipid, steroid, fatty acid, oligonucleotide, quantum dot, FRET quencher (fluorescence resonance energy transfer quencher) or a polymer soluble or insoluble in water, and wherein R⁷ represents a hydrogen atom, an alkyl group, reporter ligand, fluorescence marker, intercalator, chelator, amino acid, amino acid amide, peptide, peptide amide, protein, carbohydrate, lipid, steroid, fatty acid, oligonucleotide, quantum dot, FRET quencher or a polymer soluble or insoluble in water, wherein each of the above mentioned groups optionally may be substituted,
wherein the groups K, L or R¹ independently of each other are substituted with at least one monohydroxy mononitrophenyl group or monohydroxy dinitrophenyl group.

2. The compound according to claim 1, wherein the compound according to formula I exhibits at least one asymmetric center, and preferably at least one group R¹ does not represent a hydrogen atom.

3. The compound according to one of the preceding claims, wherein each second, or each third, group R¹ independently of each other represents an optionally substituted alkyl, alkenyl, alkylaryl, aryl, heterocyclic or alicyclic group having up to 20 carbon atoms, and the remaining groups R¹ represent hydrogen atoms.

4. The compound according to one of the preceding claims, wherein two, three or more adjacent groups R¹ independently of each other represent optionally substituted alkyl, alkenyl, alkylaryl, aryl, heterocyclic or alicyclic groups having up to 20 carbon atoms, and the remaining groups R¹ represent hydrogen atoms.

5. The compound according to one of the claims 1 to 2, wherein each R¹ independently of each other represents an optionally substituted alkyl, alkenyl, alkylaryl, aryl, heterocyclic or alicyclic group having up to 20 carbon atoms.

6. The compound according to one of the preceding claims, wherein one or more of the groups R¹ independently of each other exhibit(s) phosphonic acid ester functions or phosphonic acid functions.

7. The compound according to one of the preceding claims, wherein all asymmetric centers exhibit the same configuration, in particular, wherein all asymmetric centers exhibit an (S)-configuration, or wherein all asymmetric centers exhibit a (R)- configuration.

8. The compound according to one of the preceding claims, wherein each R¹ independently of each other exhibits one ore more phosphonic acid ester functions or phosphonic acid functions, wherein the phosphonic acid ester functions have the formula -P(=O)(OV)₂ or -P(=O)(OV)(OH), wherein each V independently of each other represents an unsubstituted alkyl, alkenyl, alkylaryl, aryl or alicyclic group having up to 20 carbon atoms, in particular, wherein each V independently of each other represents a methyl, ethyl, cyclohexyl or benzyl group.

9. A compound containing at least two compounds according to one of the claims 1 to 8, which are connected to each other via a linker, wherein the linker preferably represents an alkyl chain, a peptide, an oligonucleotide or an oligomer that is composed of at least three units of 8-amino-3,6-dioxaoctanoic acid.

10. A composition containing at least one compound according to one of the preceding claims, in particular, a pharmaceutical composition containing at least one compound according to one of the claims 1 to 9, optionally in combination with at least one carrier, solvent or other pharmaceutical adjuvant.

11. Use of a compound or composition according to one of the claims 1 to 10 for the preparation of a medicament for the treatment or prophylaxis of mitochondrial diseases.

12. Use of a compound or composition according to one of the claims 1 to 10 for the preparation of an agent for the diagnosis of mitochondrial properties or mitochondrial diseases.

13. Use according to one of the claims 11 to 12, wherein the mitochondrial disease is a hereditary disease.

14. Use according to one of the claims 11 to 13 for the treatment or prophylaxis of cancer, diabetes, Parkinson's disease, the phenomena of aging, or arteriosclerosis.

15. Use of a compound or composition according to one of the claims 1 to 10 for the preparation of a medicament for the prevention of transcription of the mtDNA in cells or in living organisms, or for the preparation of a medicament for the prevention of translation of the mtRNA in cells or in living organisms.

## Revendications

1. Composé de formule I, 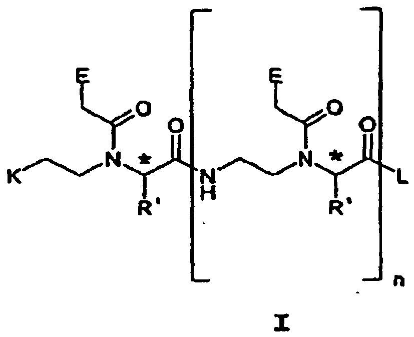 dans laquelle
n est un nombre entier entre 0 et 35,
chaque groupe E est indépendamment des autres un atome H, un radical phényle substitué ou non substitué, un hétérocycle substitué ou non substitué, une nucléobase substituée éventuellement avec des groupes protecteurs, ou un composé d'intercalation dans l'ADN,
chaque groupe R¹ est indépendamment des autres un atome H ou une chaîne latérale d'un acide aminé naturel ou non naturel, ou un radical alkyle, alcényle, alkylaryle, aryle, hétérocyclyle ou alicyclyle, éventuellement substitué, comprenant jusqu'à 20 atomes C, où au moins un des radicaux alkyle, alcényle, alkylaryle, aryle, hétérocyclyle ou alicyclyle, éventuellement substitué, comprenant jusqu'à 20 atomes C, est substitué avec une ou plusieurs fonctions ester d'acide phosphonique ou acide phosphonique,
K est un groupe de la formule -NR²R³, -N^{⊕}R²R³R⁴,-NR²(CO)R³ ou -NR²(CS)R³, où R², R³ et R⁴ sont indépendamment les uns des autres un atome H, un radical alkyle, alcaryle, alcényle ou alcinyle, un groupe protecteur amine, un ligand rapporteur, un marqueur de fluorescence, un composé d'intercalation, un agent chélateur, un acide aminé, un peptide, une protéine, un hydrate de carbone, un lipide, un stéroïde, un acide gras, un oligonucléotide, un point quantique, un désactivateur FRET (désactivateur de transfert d'énergie par résonance en fluorescence) ou un polymère soluble dans l'eau ou insoluble, chacun des radicaux ci-dessus pouvant être éventuellement substitué,
L est un groupe de la formule -NR⁵R⁶, -NR⁵(CO)R⁶,-NR⁵(CS)R⁶, -OR⁷ ou -SR⁷, où R⁵ et R⁶ sont indépendamment l'un de l'autre un atome H, un radical alkyle, alkaryle, alcényle ou alcinyle, un ligand rapporteur, un marqueur de fluorescence, un composé d'intercalation, un agent chélateur, un acide aminé, un amide d'acide aminé, un peptide, un peptide-amide, une protéine, un hydrate de carbone, un lipide, un stérol, un acide gras, un oligonucléotide, un point quantique, un désactivateur FRET (désactivateur de transfert d'énergie par résonance en fluorescence) ou un polymère soluble dans l'eau ou insoluble , et R⁷ est un atome H, un radical alkyle, un ligand rapporteur, un marqueur de fluorescence, un composé d'intercalation, un agent chélateur, un acide aminé, un amide d'acide aminé, un peptide, un peptide-amide, une protéine, un hydrate de carbone, un lipide, un stéroïde, un acide gras, un oligonucléotide, un point quantique, un désactivateur FRET ou un polymère soluble dans l'eau ou insoluble, chacun des radicaux ci-dessus pouvant être éventuellement substitué,
les radicaux K, L ou R¹ étant indépendamment les uns des autres substitués avec au moins un radical monohydroxy-mononitro-phényle ou un radical monohydroxy-dinitro-phényle.

2. Composé selon la revendication 1, le composé de formule I présentant au moins un centre asymétrique, et, de préférence, au moins un radical R¹ n'étant pas un atome H.

3. Composé selon l'une des revendications précédentes, chaque deuxième ou chaque troisième radical R¹ étant indépendamment des autres un radical alkyle, alcényle, alkylaryle, aryle, hétérocyclyle ou alicyclyle éventuellement substitué comprenant jusqu'à 20 atomes C, et les autres radicaux R¹ étant des atomes H.

4. Composé selon l'une des revendications précédentes, deux, trois ou plus de radicaux R¹ voisins étant, indépendamment les uns des autres, des radicaux alkyles, alcényles, alkylaryles, aryles, hétérocyclyles ou alicyclyles éventuellement substitués comprenant jusqu'à 20 atomes C, et les autres radicaux R¹ étant des atomes H.

5. Composé selon l'une des revendications 1 à 2, chaque R¹ étant indépendamment des autres un radical alkyle, alcényle, alkylaryle, aryle, hétérocyclyle ou alicyclyle éventuellement substitué comprenant jusqu'à 20 atomes C.

6. Composé selon l'une des revendications précédentes, un ou plusieurs radicaux R¹ présentant indépendamment les uns des autres des fonctions ester d'acide phosphonique ou des fonctions acide phosphonique.

7. Composé selon l'une des revendications précédentes, tous les centres asymétriques présentant la même configuration, tous les centres asymétriques présentant notamment une configuration (S), ou tous les centres asymétriques présentant une configuration (R).

8. Composé selon l'une des revendications précédentes, chaque R¹ présentant, indépendamment des autres, une ou plusieurs fonctions ester d'acide phosphonique ou acide phosphonique, les fonctions ester d'acide phosphonique possédant la formule -P(=O)(OV)₂ ou -P(=O)(OV)(OH), chaque V étant, indépendamment des autres, un radical alkyle, alcényle, alkylaryle, aryle ou alicyclyle non substitué comprenant jusqu'à 20 atomes C, chaque V étant, notamment, indépendamment des autres, un radical méthyle, éthyle, cyclohexyle ou benzyle.

9. Composé contenant au moins deux composés selon l'une des revendications 1 à 8, lesquels étant reliés ensemble par un agent de liaison,
l'agent de liaison étant de préférence une chaîne alkyle, un peptide, un oligonucléotide ou un oligomère qui est constitué d'au moins trois unités d'acide 8-amino-3,6-dioxaoctane.

10. Composition, qui contient au moins un composé selon l'une des revendications précédentes, notamment, composition pharmaceutique qui contient au moins un composé selon l'une des revendications 1 à 9, éventuellement en combinaison avec au moins un véhicule, un solvant ou un autre produit auxiliaire pharmaceutique.

11. Utilisation d'un composé ou d'une composition selon l'une des revendications 1 à 10 pour la préparation d'un médicament destiné au traitement ou à la prophylaxie de maladies mitochondriales.

12. Utilisation d'un composé ou d'une composition selon l'une des revendications 1 à 10 pour la préparation d'un moyen destiné au diagnostic de propriétés mitochondriales ou de maladies mitochondriales.

13. Utilisation selon l'une des revendications 11 à 12, la maladie mitochondriale étant une maladie héréditaire.

14. Utilisation selon l'une des revendications 11 à 13 pour le traitement ou la prophylaxie du cancer, du diabète, de la maladie de Parkinson, des phénomènes de vieillissement, ou de l'artériosclérose.

15. Utilisation d'un composé ou d'une composition selon l'une des revendications 1 à 10 pour la préparation d'un médicament destiné à l'empêchement de la transcription de l'ADN mt dans des cellules ou organismes vivants, ou pour la préparation d'un médicament destiné à l'empêchement de la traduction de l'ADN mt dans des cellules ou organismes vivants.
